(19) **Europäisches Patentamt / European Patent Office / Office européen des brevets**

(11) **EP 4 063 477 A1**

(12) **EUROPEAN PATENT APPLICATION**
published in accordance with Art. 153(4) EPC

(43) Date of publication:
**28.09.2022 Bulletin 2022/39**

(21) Application number: **20889386.7**

(22) Date of filing: **17.11.2020**

(51) International Patent Classification (IPC):
**C12M 1/00** (2006.01)　　**C12M 3/00** (2006.01)
**C12N 5/071** (2010.01)　　**C12N 5/0735** (2010.01)
**C12N 5/074** (2010.01)　　**C12N 5/077** (2010.01)
**C12N 5/079** (2010.01)　　**C12N 5/09** (2010.01)
**C12N 1/00** (2006.01)　　**C12Q 1/02** (2006.01)
**C12N 11/02** (2006.01)

(52) Cooperative Patent Classification (CPC):
**C12M 1/00; C12M 3/00; C12N 1/00; C12N 5/06;
C12N 11/02; C12Q 1/02**

(86) International application number:
**PCT/JP2020/042861**

(87) International publication number:
**WO 2021/100718 (27.05.2021 Gazette 2021/21)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **21.11.2019 JP 2019210649
08.09.2020 JP 2020150816**

(71) Applicants:
• **The Japan Wool Textile Co., Ltd.
Kobe-shi, Hyogo 650-0037 (JP)**
• **The University of Tokyo
Bunkyo-ku, Tokyo 113-8654 (JP)**

(72) Inventors:
• **SAWADA Kohei
Tokyo 113-8654 (JP)**
• **BABA Atsushi
Tokyo 113-8654 (JP)**
• **SAOTOME Toshiki
Kakogawa-shi, Hyogo 675-0053 (JP)**
• **SHIMADA Naoki
Kakogawa-shi, Hyogo 675-0053 (JP)**

(74) Representative: **Dehns
St. Bride's House
10 Salisbury Square
London EC4Y 8JD (GB)**

(54) **CELL AGGREGATE, PRODUCTION METHOD FOR CELL AGGREGATE, PRODUCTION KIT FOR CELL AGGREGATE, AND CHEMICAL COMPOUND EVALUATION METHOD USING CELL AGGREGATE**

(57)　The present invention relates to a multicellular construct that includes cells and a scaffold. The scaffold is constituted by a layered composite that comprises a gelatin nonwoven containing gelatin as a main component, and a gelatin film containing gelatin as a main component and layered on one surface of the gelatin nonwoven, and the cells are present in at least one of a region on the surface of the gelatin nonwoven and a region inside the nonwoven. The multicellular construct can be produced by arranging the scaffold in a swollen state inside the culture vessel whose inner surface is in the dry state such that the gelatin film of the scaffold is in contact with the inner bottom surface of the culture vessel, dripping a cell suspension onto the gelatin nonwoven of the scaffold, and then culturing the cells. This makes it possible to provide a multicellular construct with high seeding efficiency in which desorption of cells from a scaffold is suppressed, a method for manufacturing the same, a kit for producing the same, and a method for evaluating a compound using the same.

EP 4 063 477 A1

**Description**

Technical Field

The present invention relates to a multicellular construct (aggregate) with high seeding efficiency in which desorption of cells from a scaffold is suppressed, a method for manufacturing the same, a kit for producing the same, and a method for evaluating a compound using the same.

Background Art

Cells interact three-dimensionally with one another in vivo, and it is known that the functions of cells are enhanced by allowing the cells to interact three-dimensionally with one another even in vitro. A cell culture scaffold constituted by a fiber sheet provides a site for culturing cells three-dimensionally and promotes interactions thereof with one another, and has thus been expected to be applied to large-scale cell culture, a medical device, cell transplantation treatment, drug safety evaluation, a disease model, and the like. In particular, a fiber sheet made of a biocompatible polymer is favorably used as a medical scaffold and a cell culture scaffold. For example, Patent Document 1 discloses that a support such as gauze or sponge on which nanofibers have been formed of biopolymers such as gelatin, collagen, and cellulose is used as a culture matrix. Patent Document 2 discloses that a biocompatible long-fiber nonwoven obtained by welding biocompatible long fibers with an average fiber diameter of 1 to 70 $\mu$m at some of fiber cross-points thereof is used as a cell culture scaffold.

Citation List

Patent Documents

Patent Document 1: WO 2014/196549
Patent Document 2: WO 2018/235745

Summary of Invention

Problem to be Solved by the Invention

However, it is known that, when cells are seeded on a cell culture scaffold constituted by a fiber sheet, the cellular distribution varies depending on the structure of the scaffold material, such as the fiber diameter, the pore size, or the density. For example, a dense scaffold has a problem in that cells are likely to remain on the surface layer, and thus the cells may move around the edge of the scaffold and fall therefrom. On the other hand, in the case of a coarse scaffold, cells enter the inside of the scaffold, but there is a problem in that the cells may pass through the scaffold, or leak from the side surface of the scaffold and fall therefrom.

The present invention was made in order to solve the aforementioned problems and provides a multicellular construct with high seeding efficiency in which desorption of cells from a scaffold is suppressed, a method for manufacturing the same, a kit for producing the same, and a method for evaluating a compound using the same.

Means for Solving Problem

The present invention relates to a multicellular construct that includes: cells; and a scaffold, wherein the scaffold is constituted by a layered composite that includes a gelatin nonwoven containing gelatin as a main component, and a gelatin film containing gelatin as a main component and layered on one surface of the gelatin nonwoven, and the cells are present in at least one of a region on the surface of the gelatin nonwoven and a region inside the gelatin nonwoven.

The present invention also relates to a method for manufacturing the multicellular construct, including: a step of preparing a scaffold constituted by a layered composite that includes a gelatin nonwoven containing gelatin as a main component, and a gelatin film containing gelatin as a main component and layered on one surface of the gelatin nonwoven, and a culture vessel whose inner surface has not undergone a hydrophilization treatment; a step of arranging the scaffold in a swollen state inside the culture vessel whose inner surface is in a dry state with the gelatin film of the scaffold being in contact with an inner bottom surface of the culture vessel; and a step of dripping a cell suspension on the gelatin nonwoven of the scaffold to start culturing.

The present invention also relates to a method for evaluating a compound by evaluating characteristics of the compound, including: a step of delivering a compound into contact with the multicellular construct; and a step of determining whether or not physiological properties of the multicellular construct are changed due to the contact with the compound.

Effects of the Invention

The present invention can provide a multicellular construct with high seeding efficiency in which desorption of cells from a scaffold is suppressed.

With the manufacturing method according to the present invention, it is possible to produce a multicellular construct at high seeding efficiency while suppressing desorption of cells from a scaffold.

The production kit according to the present invention can be used to produce a multicellular construct at high seeding efficiency while suppressing desorption of cells from a scaffold.

With the present invention, the three-dimensionally cultured multicellular construct can be used to effectively evaluate the properties of a compound.

Brief Description of Drawings

[FIG. 1] FIG. 1 is a scanning electron micrograph (100-fold magnification) of a scaffold (layered composite) used in an example of the present invention.

[FIG. 2] FIG. 2 is a scanning electron micrograph (100-fold magnification) of a scaffold (layered composite) used in another example of the present invention.

[FIG. 3] FIG. 3 shows images of an end portion of a scaffold observed under an optical microscope (4-fold magnification) in Example 1.

FIG. 3(a) shows the result obtained after cells had been seeded.

FIG. 3(b) shows the result obtained when a liquid culture medium was added after the scaffold had been cultured for 3 hours.

[FIG. 4] FIG. 4 shows fluorescence images of viable cells in a scaffold observed in Example 1.

FIG. 4(a) is an image of the entire scaffold after cells had been seeded thereon.

FIG. 4(b) is an enlarged image of the central portion of the scaffold shown in FIG. 4(a).

FIG. 4(c) is an image of the entire scaffold (multicellular construct) taken when a liquid culture medium was added after the scaffold had been cultured for 3 hours.

FIG. 4(d) is an enlarged image of the central portion of the scaffold shown in FIG. 4(c).

FIG. 4(e) is an image of the entire scaffold (multicellular construct) taken after the scaffold had been cultured for 72 hours.

FIG. 4(f) is an enlarged image of the central portion of the scaffold shown in FIG. 4(e).

[FIG. 5] FIG. 5 shows fluorescence images of viable cells in a scaffold observed in Example 2.

FIG. 5(a) is an image of the entire scaffold after cells had been seeded thereon.

FIG. 5(b) is an enlarged image of the central portion of the scaffold shown in FIG. 5(a).

FIG. 5(c) is an image of the entire scaffold (multicellular construct) taken when a liquid culture medium was added after the scaffold had been cultured for 3 hours.

FIG. 5(d) is an enlarged image of the central portion of the scaffold shown in FIG. 5(c).

FIG. 5(e) is an image of the entire scaffold (multicellular construct) taken after the scaffold had been cultured for 72 hours.

FIG. 5(f) is an enlarged image of the central portion of the scaffold shown in FIG. 5(e).

[FIG. 6] FIG. 6 shows images of an end portion of a scaffold observed under an optical microscope (4-fold magnification) in Example 3.

FIG. 6(a) shows the result obtained after cells had been seeded.

FIG. 6(b) shows the result obtained when a liquid culture medium was added after the scaffold had been cultured for 3 hours.

[FIG. 7] FIG. 7 shows fluorescence images of viable cells in a scaffold observed in Example 3.

FIG. 7(a) is an image of the entire scaffold after cells had been seeded thereon.

FIG. 7(b) is an enlarged image of the central portion of the scaffold shown in FIG. 7(a).

FIG. 7(c) is an image of the entire scaffold (multicellular construct) taken when a liquid culture medium was added after the scaffold had been cultured for 3 hours.

FIG. 7(d) is an enlarged image of the central portion of the scaffold shown in FIG. 7(c).

FIG. 7(e) is an image of the entire scaffold (multicellular construct) taken after the scaffold had been cultured for 72 hours.

FIG. 7(f) is an enlarged image of the central portion of the scaffold shown in FIG. 7(e).

[FIG. 8] FIG. 8 shows fluorescence images of viable cells in a scaffold observed in Example 4.

FIG. 8(a) is an image of the entire scaffold after cells had been seeded thereon.

FIG. 8(b) is an enlarged image of the central portion of the scaffold shown in FIG. 8(a).

FIG. 8(c) is an image of the entire scaffold (multicellular construct) taken when a liquid culture medium was added after the scaffold had been cultured for 3 hours.

FIG. 8(d) is an enlarged image of the central portion of the scaffold shown in FIG. 8(c).

FIG. 8(e) is an image of the entire scaffold (multicellular construct) taken after the scaffold had been cultured for 72 hours.

FIG. 8(f) is an enlarged image of the central portion of the scaffold shown in FIG. 8(e).

[FIG. 9] FIG. 9 shows images of an end portion of a scaffold observed under an optical microscope (4-fold magnification) in Comparative Example 1.

FIG. 9(a) shows the result obtained after cells had been seeded.

FIG. 9(b) shows the result obtained when a liquid culture medium was added after the scaffold had been cultured for 3 hours.

[FIG. 10] FIG. 10 shows images of an end portion of a scaffold observed under an optical microscope (4-fold magnification) in Comparative Example 2.

FIG. 10(a) shows the result obtained after cells had been seeded.

FIG. 10(b) shows the result obtained when a liquid culture medium was added after the scaffold had been cultured for 3 hours.

[FIG. 11] FIG. 11 shows images illustrating viable cell distribution in the scaffold observed after the scaffold had been cultured for 72 hours in Example 1.

FIG. 11(a) shows the result from the observation of the surface on a gelatin nonwoven side.

FIG. 11(b) shows the result from a portion that is 100 $\mu$m away from the surface of the gelatin nonwoven in the thickness direction.

FIG. 11(c) shows the result from a portion that is 200 $\mu$m away from the surface of the gelatin nonwoven in the thickness direction.

FIG. 11(d) shows the result from a portion that is 200 $\mu$m away from the surface of a gelatin film in the thickness direction.

FIG. 11(e) shows the result from a portion that is 100 $\mu$m away from the surface of a gelatin film in the thickness direction.

FIG. 11(f) shows the result from the observation of the surface on the gelatin film side.

[FIG. 12] FIG. 12 shows images illustrating viable cell distribution in the scaffold observed after the scaffold had been cultured for 72 hours in Example 2.

FIG. 12(a) shows the result from the observation of the surface on a gelatin nonwoven side.

FIG. 12(b) shows the result from a portion that is 100 $\mu$m away from the surface of the gelatin nonwoven in the thickness direction.

FIG. 12(c) shows the result from a portion that is 200 $\mu$m away from the surface of the gelatin nonwoven in the thickness direction.

FIG. 12(d) shows the result from a portion that is 200 $\mu$m away from the surface of a gelatin film in the thickness direction.

FIG. 12(e) shows the result from a portion that is 100 $\mu$m away from the surface of a gelatin film in the thickness direction.

FIG. 12(f) shows the result from the observation of the surface on the gelatin film side.

[FIG. 13] FIG. 13 shows the results of calcium ion imaging observation on a multicellular construct on the fourth day of the culture in Example 5.

FIG. 13(a) is a bright field image of the multicellular construct.

FIG. 13(b) is a fluorescence image of calcium ions in the cells observed at a predetermined time.

FIG. 13(c) is a fluorescence image of calcium ions in the cells observed 0.7 seconds after the image shown in FIG. 13(b) had been taken.

FIG. 13(d) is a graph obtained by plotting the fluorescence intensities at measurement points A and B versus time.

[FIG. 14] FIG. 14 shows the results illustrating the response of the multicellular construct against isoproterenol on the fourth day of the culture in Example 5.

[FIG. 15] FIG. 15 shows the results of calcium ion imaging observation on a multicellular construct on the fourth day of the culture in Example 6.

FIG. 15(a) is a bright field image of the multicellular construct.

FIG. 15(b) is a fluorescence image of intracellular calcium ions observed at a predetermined time.

FIG. 15(c) is a fluorescence image of intracellular calcium ions observed 0.7 seconds after the image shown in FIG. 15(b) had been taken.

FIG. 15(d) is a graph obtained by plotting the fluorescence intensities at measurement points A and B versus time.

[FIG. 16] FIG. 16 shows the results illustrating the response against E-4031 on the fourth day of the culture in Example 6.

FIG. 16(a) is a fluorescence image of intracellular calcium ions observed 120 minutes after the addition of E-4031.

FIG. 16(b) is a fluorescence image of intracellular calcium ions observed 0.4 seconds after the image shown in FIG. 16(a) had been taken.

FIG. 16(c) is a graph obtained by plotting the fluorescence intensities at measurement points A and B versus time.

[FIG. 17] FIG. 17 shows the results of fluorescence staining of a cardiomyocyte multicellular construct in Example 7.

FIG. 17(a) shows a stained image of cell nuclei and actin filaments on the third day of the culture.

FIG. 17(b) shows an immunostained image of $\alpha$-actanin on the third day of the culture.

FIG. 17(c) is a partially enlarged image of FIG. 17(b).

FIG. 17(d) shows a stained image of cell nuclei and actin filaments on the seventh day of the culture.

FIG. 17(e) shows an immunostained image of $\alpha$-actinin on the seventh day of the culture.

FIG. 17(f) is a partially enlarged image of FIG. 17(e).

[FIG. 18] FIG. 18 shows graphs illustrating the responses against various compounds in Example 7.

FIG. 18(a) shows the calcium ion signal waveforms before and after the addition of isoproterenol.

FIG. 18(b) shows the beating rates for various concentrations of isoproterenol.

FIG. 18(c) shows the calcium ion signal waveforms before and after the addition of E-4031.

FIG. 18(d) shows the beating rates for various concentrations of E-4031.

FIG. 18(e) shows the calcium ion signal waveforms before and after the addition of verapamil.

FIG. 18(f) shows the beating rates for various concentrations of verapamil.

[FIG. 19] FIG. 19 shows graphs illustrating the responses against various compounds in Example 7.

FIG. 19(a) shows the contractile forces before and after the addition of isoproterenol.

FIG. 19(b) shows the contraction-relaxation rates before and after the addition of isoproterenol.

FIG. 19(c) shows the contractile forces before and after the administration of E-4031.

FIG. 19(d) shows the contraction-relaxation rates before and after the addition of E-4031.

FIG. 19(e) shows the contractile forces before and after the addition of verapamil.

FIG. 19(f) shows the contraction-relaxation rates before and after the addition of verapamil.

[FIG. 20] FIG. 20 shows graphs illustrating the responses against various compounds (calcium ion signal waveforms) in Example 8.

FIG. 20(a) shows the results from the case of azithromycin.

FIG. 20(b) shows the results from the case of chloroquine.

FIG. 20(c) shows the results from the case of hydroxychloroquine.

FIG. 20(d) shows the results from the case of a combination of azithromycin (3 $\mu$M) and chloroquine.

FIG. 20(e) shows the results from the case of a combination of azithromycin (3 $\mu$M) and hydroxychloroquine.

[FIG. 21] FIG. 21 shows graphs illustrating the responses against various compounds (contractile forces) in Example 8.

FIG. 21(a) shows the results from the case of azithromycin.

FIG. 21(b) shows the results from the case of chloroquine.

FIG. 21(c) shows the results from the case of hydroxychloroquine.

FIG. 21(d) shows the results from the case of azithromycin (3 $\mu$M) and chloroquine.

FIG. 21(e) shows the results from the case of azithromycin (3 $\mu$M) and hydroxychloroquine.

[FIG. 22] FIG. 22 shows graphs illustrating the responses against various compounds in Example 9.

[FIG. 23] FIG. 23 is a schematic explanatory diagram of a scaffold manufacturing apparatus used in an example of the present invention.

Disclosure of Invention

The inventors of the present invention carried out extensive research in order to solve the aforementioned problems. As a result, they found that a multicellular construct with high seeding efficiency in which desorption of cells from a scaffold is suppressed is obtained by using a scaffold constituted by a layered composite that includes a gelatin nonwoven containing gelatin as a main component, and a gelatin film containing gelatin as a main component and layered on one surface of the gelatin nonwoven, and allowing cells to be present in at least one of a region on the surface of the gelatin nonwoven and a region inside the nonwoven, preferably both of the regions.

It was surprisingly found that, especially by using a scaffold constituted by a layered composite that includes a gelatin nonwoven containing gelatin as a main component, and a gelatin film containing gelatin as a main component and layered on one surface of the gelatin nonwoven, and a (non-hydrophilic) culture vessel whose inner surface (that includes an inner bottom surface and an inner side surface) has not undergone a hydrophilization treatment, arranging the scaffold in a swollen state inside the culture vessel whose inner surface is in the dry state such that the gelatin film of the scaffold is in contact with the inner bottom surface of the culture vessel, and dripping a cell suspension onto the gelatin nonwoven of the scaffold, the cell suspension remains on the scaffold, the leakage of the cells to the edge of the scaffold is suppressed (i.e., desorption of the cells from the scaffold is suppressed), and the seeding efficiency is enhanced. It is presumed that, since the hydrophilicity (wettability) of the scaffold constituted by a layered composite that includes a gelatin nonwoven and a gelatin film is higher than the hydrophilicity (wettability) of the inner surface of the culture vessel, the cell suspension does not flow toward the culture vessel from the scaffold, and thus the cells do not desorb from the scaffold. Also, if cell adhesion is promoted by performing preculture for a predetermined period of time in a state in which the cells remain inside the scaffold and/or on the surface of the scaffold, and then an additional liquid culture medium is added to immerse the scaffold in the liquid culture medium, the cells remain on the scaffold and do not desorb from the scaffold.

On the other hand, in the case of a (hydrophilic) culture vessel whose inner surface such as the inner bottom surface has undergone a hydrophilization treatment in order to enhance the adhesiveness to cells, when a cell suspension is dripped onto the scaffold, the cell suspension flows along the periphery of scaffold, and thus the cells fall from the scaffold.

In general, in the case of proliferative cells, a cell proliferation period that is longer than or equal to a week is needed to increase the cell density in a scaffold. In the case of non-proliferative cells, if the seeding efficiency is poor, it is difficult to increase the cell density in a scaffold. However, with the above-described seeding method, the seeding efficiency is high, and thus high-density seeding is possible. Accordingly, cells can be cultured at high density from the beginning of the culture.

Therefore, a multicellular construct that is three-dimensionally cultured at high density from the beginning of the culture is obtained, and this multicellular construct can be used to effectively evaluate the properties of a compound.

The term "swelling" as used in the present invention means swelling a scaffold to saturation with one or more liquids selected from the group consisting of water, a buffer, and a liquid culture medium.

Both the gelatin nonwoven and the gelatin film contain gelatin as a main component. The term "main component" as used in the present invention means that gelatin is contained in an amount of 90 mass% or more. Other components contained in an amount of 10 mass% or less may include another biocompatible polymer, a cross-linking agent, a drug, a plasticizer, and other additives as needed. Gelatin may be contained in an amount of substantially 100 mass%. The scaffold according to the present invention contains gelatin, which is highly safe and excellent in bioabsorbability, as a main component, and can thus be favorably used as a scaffold for regenerative treatment to be transplanted into a living organism, a three-dimensional cell tissue needed for cell research and drug discovery research, and the like.

There is no particular limitation on the animal species and the sites from which collagen used as a raw material of the gelatin is derived. Collagen may be derived from, for example, spinal animals including fish. Also, collagen derived from various organs and tissues such as the dermis, the ligament, the tendon, the bone, and the cartilage can be used as appropriate. There is also no particular limitation on a method for preparing gelatin from collagen, and examples thereof include acid treatment, alkali treatment, and enzymatic treatment. There is also no particular limitation on the molecular weight of the gelatin, and gelatin with a molecular weight selected from various molecular weights as appropriate can be used. One type of gelatin may be used, or two or more types of gelatin may be used together.

The gelatin is not particularly limited but has appropriate flexibility and hardness. The jelly strength thereof is preferably 100 g or more and 400 g or less, and more preferably 150 g or more and 360 g or less, from the viewpoint of improving the handleability of the scaffold. In the present invention, the jelly strength is measured in conformity with JIS K 6503. Commercially available gelatin may be used.

Examples of the other biocompatible polymer include, but are not particularly limited to, natural polymers and synthetic polymers. Examples of the natural polymers include proteins and polysaccharides. Examples of the proteins include collagen, fibronectin, fibrinogen, laminin, and fibrin. Examples of the polysaccharides include: natural polymers such as chitosan, calcium alginate, heparan sulfate, chondroitin sulfate, hyaluronic acid, heparin, starch, gellan gum, agarose, guar gum, xanthan gum, carrageenan, pectin, locust bean gum, tamarind gum, and diutan gum; and derivatives of natural polymers such as carboxymethyl cellulose. Examples of the synthetic polymers include: non-absorbable synthetic polymers such as polyethylene glycol, polyethylene glycol, polyethylene terephthalate, polyvinyl alcohol, thermoplastic elastomers, polypropylene, polyethylene, polystyrene, polymethyl methacrylate, polycarbonates, dimethylpolysiloxane, cycloolefin polymers, and amorphous fluororesins; and bioabsorbable polymers such as polylactic acid, polyglycolic acid, polycaprolactone, and polydioxanone. One type or two or more types of the above-described other biocompatible polymers may be used.

After swelling, gelatin fibers included in the gelatin nonwoven preferably have an average fiber diameter of 2 $\mu$m or more and 400 $\mu$m or less, more preferably 5 $\mu$m or more and 200 $\mu$m or less, even more preferably 10 $\mu$m or more and 100 $\mu$m or less, and particularly preferably 15 $\mu$m or more and 45 $\mu$m or less. If the gelatin fibers have an average fiber diameter within the range mentioned above, cells that have been seeded on the surface of the gelatin nonwoven in the scaffold are likely to enter the scaffold and to be uniformly distributed inside the scaffold. The "average fiber diameter after swelling" in the present invention means the average value of the diameters of fifty fibers that are randomly selected from the gelatin nonwoven in the swollen scaffold.

It is preferable that the gelatin fibers included in the gelatin nonwoven are welded to one another at some of fiber cross-points thereof. Such partial welding can be achieved as described later by, for example, depositing gelatin fibers that are blown by a pressure fluid and do not completely solidify during the manufacturing of the scaffold, but there is no particular limitation thereto. The gelatin nonwoven has a bridge structure due to this partial welding and can thus be molded into a desired shape, and the molding stability thereof can be increased. Since the fibers are welded to one another at some of the fiber cross-points, the gelatin nonwoven is not weakened when getting wet with water. In the gelatin nonwoven, the fibers may be welded to one another at some of the fiber cross-points or at all of the fiber cross-points.

The gelatin nonwoven preferably has a thickness of 0.1 mm or more, more preferably 0.2 mm or more, even more preferably 0.3 mm or more, and particularly preferably 0.4 mm or more, from the viewpoint of, for example, enhancing the handleability and facilitating entering of the cells, but there is no particular limitation thereto. Also, the gelatin nonwoven preferably has a thickness of 2 mm or less, more preferably 1.5 mm or less, even more preferably 1 mm or less, and particularly preferably 0.7 mm or less, from the viewpoint of, for example, enhancing the viability of cells during three-dimensional culture, but there is no particular limitation thereto.

The gelatin nonwoven preferably has a basis weight of 10 g/m$^2$ or more, more preferably 25 g/m$^2$ or more, and even more preferably 50 g/m$^2$ or more, from the viewpoint of, for example, suppressing desorption of cells, but there is no particular limitation thereto. Also, the gelatin nonwoven preferably has a basis weight of 600 g/m$^2$ or less, more preferably 500 g/m$^2$ or less, and even more preferably 400 g/m$^2$ or less, from the viewpoint of, for example, facilitating entering of the cells and enhancing the viability of cells during three-dimensional culture, but there is no particular limitation thereto.

The gelatin nonwoven preferably has a pore diameter of 20 $\mu$m or more, more preferably 30 $\mu$m or more, and even more preferably 40 $\mu$m or more, from the viewpoint of, for example, facilitating entering of the cells and enhancing the viability of cells during three-dimensional culture, but there is no particular limitation thereto. Also, the gelatin nonwoven preferably has a pore diameter of 250 $\mu$m or less, more preferably 200 $\mu$m or less, and even more preferably 180 $\mu$m or less, from the viewpoint of, for example, suppressing desorption of cells, but there is no particular limitation thereto. In the present invention, the pore diameter of the gelatin nonwoven can be calculated using Computational Expression (1) below based on the Wrotnowski's hypothesis.

[Mathematical Formula 1]

Computational Expression 1

$$D = \frac{42.5}{cos45\sqrt{\dfrac{W}{F}}} - f$$

$$f = 11.9 \times \sqrt{\dfrac{F}{\rho}}$$

D: Pore diameter ($\mu$m)
W: Apparent density of nonwoven (g/cm$^3$)
F: Fineness (d)
f: Fiber diameter ($\mu$m)
$\rho$: Fiber density (g/cm$^3$)

It is preferable that the gelatin film is arranged on one surface of the gelatin nonwoven and is welded to some of the gelatin fibers included in the gelatin nonwoven. Such partial welding can be achieved as described later by, for example, depositing gelatin fibers that are blown by a pressure fluid and do not completely solidify on the gelatin film during the manufacturing of the scaffold, but there is no particular limitation thereto. The gelatin nonwoven and the gelatin film are integrated through this partial welding, which inhibits cells from passing through the scaffold and desorbing from the scaffold. Due to the integration of the gelatin nonwoven and the gelatin film, a multicellular construct can be easily separated from a culture vessel. Also, damage to the cells is reduced, so that favorable engraftment in a recipient is achieved.

The gelatin film preferably has a thickness of 0.5 μm or more, more preferably 0.6 μm or more, even more preferably 0.7 μm or more, and particularly preferably 0.8 μm or more, from the viewpoint of, for example, the convenience and the handleability. Also, the gelatin film preferably has a thickness of 10 μm or less, more preferably 8 μm or less, even more preferably 6 μm or less, even more preferably 4 μm or less, and particularly preferably 2 μm or less, from the viewpoint of, for example, enhancing the integrity with the gelatin nonwoven and enhancing the efficiency of three-dimensional cell culture.

The gelatin film is preferably a non-porous film, but may be provided with micropores having such a size that cells do not pass therethrough, such as micropores having a pore diameter of about 10 μm or less or 5 μm or less.

A ratio Tf/Tn of a thickness Tf of the gelatin film to a thickness Tn of the gelatin nonwoven is preferably $7.5 \times 10^{-3}$ or less. With this configuration, warping of the swollen layered composite is suppressed, and the cell suspension is more likely to remain on the scaffold. Furthermore, even in the case where the layered composite is swollen, the gelatin film is likely to follow the gelatin nonwoven, and thus separation and breakage of the gelatin film are less likely to occur. Tf/Tn above is more preferably $7.0 \times 10^{-3}$ or less, and even more preferably $6.0 \times 10^{-3}$ or less. Also, Tf/Tn above is preferably $1.0 \times 10^{-3}$ or more, and more preferably $1.5 \times 10^{-3}$ or more, from the viewpoint that the separation and breakage of the gelatin film are likely to be suppressed.

The compressive deformation rate of the layered composite to which 1.0 kPa of compressive stress is applied after swelling to saturation with water (such a compressive deformation rate is also referred to simply as a "compressive deformation rate" hereinafter) is preferably 40% or less, more preferably 35% or less, and even more preferably 30% or less, from the viewpoint of, for example, maintaining the strength of the layered composite during cell culture and enhancing the viability of cells during three-dimensional culture, but there is no particular limitation thereto. The "saturation" above means a state in which water is contained to the maximum extent, and the water content remains at a certain level and does not further increase. In this specification, the thickness of the layered composite to which no load is applied after swollen to saturation with water is taken as H1, the thickness of the layered composite to which 1.0 kPa of compressive stress is applied after swollen to saturation with water is taken as H2, and the compressive deformation rate is calculated using the following formula. A compression test is performed as described later.

$$\text{Compressive deformation rate (\%)} = 100 - \{(\text{H2/H1}) \times 100\}$$

It is preferable that each of the gelatin nonwoven and the gelatin film is cross-linked from the viewpoint that the shapes thereof are likely to be maintained during cell culture and three-dimensional cell culture is effectively performed. Specifically, cross-linking can be performed as described later.

In one or more embodiments of the present invention, the layered composite that includes the gelatin nonwoven and the gelatin film may be coated with a cell adhesion factor, a cell inducing factor, a cell growth factor, a substance serving as a nutrient source or energy source for cells, a substance that suppresses or enhances the cell functions, or the like. There is no particular limitation on the cell adhesion factor, and an example thereof is fibronectin. Coating the layered composite with a cell adhesion factor makes it more likely that cells remain on the scaffold. Examples of the substance serving as a nutrient source or energy source for cells include, but are not particularly limited to, ATP, pyruvic acid, and glutamine. Also, in one or more embodiments of the present invention, the layered composite that includes the gelatin nonwoven and the gelatin film may be immersed in a solution containing physiologically active substances such as a cell inducing factor and a cell growth factor and be impregnated with these components. In the cell culture process, these physiologically active substances are gradually released from the layered composite, thus making it possible to promote the cell culture.

There is no particular limitation on the way to produce the scaffold, but it is preferable to produce the scaffold as follows from the viewpoint of suppressing the generation of impurities, preventing the contamination of products, and integrating a gelatin nonwoven and a gelatin film without using a binder component and a thermocompression bonding means: a spinning solution containing gelatin is discharged into the air from a nozzle discharge outlet, a pressure fluid is sprayed forward from a fluid spout that is located on the rear side relative to the nozzle discharge outlet and is not in contact with the nozzle discharge outlet, the pressure fluid is allowed to accompany the discharged spinning solution to form fibers, a gelatin nonwoven is formed by accumulating the formed fibers on a gelatin film containing gelatin as a main component, and thus a layered composite that includes the gelatin film and the gelatin nonwoven is obtained.

The gelatin film is not particularly limited, and can be produced using a known film manufacturing method. For example, the gelatin film can be produced by applying a gelatin solution on the surface of a substrate and then drying the substrate. Examples of the substrate include a polyethylene terephthalate film (PET film), a glass plate, a polystyrene sheet, and a fluororesin sheet. The PET film, the glass plate, the polystyrene sheet, the fluororesin sheet, and the like may undergo a water-repellent treatment.

The gelatin solution can be obtained by dissolving, in a solvent, only gelatin, or gelatin and another biocompatible polymer that can be used as the other component described above as needed. Examples of the solvent include water, alcohols such as ethanol, 1-propanol, 2-propanol, and glycerin, and amides such as dimethylformamide and dimethylacetamide. These solvents may be used alone or as a mixture of two or more. In particular, it is preferable to use water such as distilled water, pure water, ultrapure water, or ion-exchanged water from the viewpoint of handleability. Since gelatin is water-soluble, an aqueous solution thereof can be used to form a film, and thus the safety in a living organism is increased.

The concentration of the gelatin is not particularly limited, but is preferably 0.1 mass% or more and 35 mass% or less, more preferably 1 mass% or more and 30 mass% or less, and even more preferably 3 mass% or more and 20 mass% or less, with respect to 100 mass% of the gelatin solution, from the viewpoint of, for example, the ability to form a film and the ability to flow and spread. The dissolution temperature (temperature of the solvent) is preferably 10°C or higher and 90°C or lower, more preferably 20°C or higher and 80°C or lower, and even more preferably 30°C or higher and 70°C or lower. After the gelatin has been dissolved in the solvent, foreign matter, dirt, and the like may be removed through filtration as needed. Thereafter, dissolved air may also be removed through reduced-pressure defoaming or vacuum defoaming as needed. The degree of vacuum during the reduced-pressure defoaming is preferably 5 kPa or more and 30 kPa or less from the viewpoint of efficiently removing gas (bubbles).

The above-mentioned drying is not particularly limited, and can be, for example, natural drying, drying by heating, reduced-pressure drying (vacuum drying), forced exhaust drying, forced circulation convection, or the like. Specifically, the drying temperature may be, for example, -40°C or higher and 90°C or lower, 0°C or higher and 60°C or lower, or 10°C or higher and 40°C or lower. The drying time may be, for example, within a range of 1 hour to 200 hours, and is preferably within a range of 3 hours to 100 hours and more preferably within a range of 5 hours to 48 hours.

The above-mentioned melt blowing method is performed as follows: a spinning solution containing gelatin is discharged from a nozzle discharge outlet, a pressure fluid is sprayed forward from a fluid spout that is located on the rear side relative to the nozzle discharge outlet and is not in contact with the nozzle discharge outlet, the pressure fluid is allowed to accompany the discharged spinning solution to directly form fibers in a dry process, and a nonwoven is formed by accumulating the formed gelatin fibers on a gelatin film. Therefore, generation of contamination (impurities) is prevented, and the product can be hygienically manufactured. When accumulated (deposited), the spun fibers are layered with moisture being contained therein. Therefore, the fibers are welded or tangled together and are thus integrated, while the gelatin fibers included in the nonwoven are welded to the gelatin film and thus the gelatin nonwoven and the gelatin film are integrated. The density of the nonwoven can be easily changed by changing the fiber collection distance when depositing the fibers.

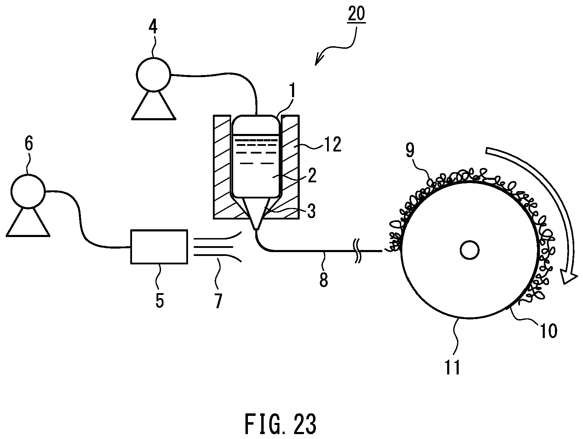

FIG. 23 is a schematic explanatory diagram of a scaffold manufacturing apparatus used in an example of the present invention. In a scaffold manufacturing apparatus 20, a spinning solution 2 containing gelatin that is contained in a warming tank 1 is discharged into the air from a nozzle discharge outlet 3. A compressor 4 is used to apply a predetermined pressure to the warming tank 1. A heat-insulating container is numbered 12.

A pressure fluid 7 is sprayed forward from a fluid spout 5 that is located on the rear side relative to the nozzle discharge outlet 3 and is not in contact with the nozzle discharge outlet 3. A compressor 6 supplies pressure fluid (e.g., compressed air) to the fluid spout 5. The distance between the fluid spout 5 and the nozzle discharge outlet 3 is preferably 5 to 30 mm.

The pressure fluid 7 accompanies the discharged spinning solution to form gelatin fibers 8. The gelatin fibers 8 are deposited on a gelatin film 10 arranged on a winding roll 11 to form a gelatin nonwoven 9. At this time, the deposited fibers contain moisture or do not completely solidify. Therefore, the fibers that are in contact with one another at some or all of the fiber cross-points are welded to one another, while the gelatin fibers included in the nonwoven are welded to the gelatin film and thus the gelatin nonwoven and the gelatin film are integrated. Note that another collection means such as a net may also be used instead of the winding roll.

First, the spinning solution is prepared by dissolving, in a solvent (preferably water), only gelatin, or gelatin and another biocompatible polymer that can be used as the other component described above as needed. The dissolution temperature (temperature of the solvent such as water) is preferably 20°C or higher and 90°C or lower, and more preferably 40°C or higher and 90°C or lower. After the gelatin has been dissolved in the solvent such as water, foreign matter, dirt, and the like may be removed through filtration as needed. Thereafter, dissolved air may also be removed through reduced-pressure defoaming or vacuum defoaming as needed. The degree of vacuum during the reduced-pressure defoaming is preferably 5 kPa or more and 30 kPa or less from the viewpoint of efficiently removing gas (bubbles). Since gelatin is water-soluble, an aqueous solution thereof can be used as the spinning solution to form fibers, and thus the safety in a living organism is increased. As the water, for example, pure water, distilled water, ultrapure water, or the like can be used as appropriate. Note that, when another water-soluble biocompatible macromolecule is used as the other component, this macromolecule can be dissolved in water together with gelatin to prepare the spinning solution.

The temperature of the spinning solution is preferably 20°C or higher and 90°C or lower, and more preferably 40°C or higher and 90°C or lower. When the temperature is within the range above, the sol state of gelatin can be stably maintained. The concentration of gelatin in the gelatin aqueous solution is preferably 30 mass% or more and 55 mass% or less with respect to 100 mass% of the gelatin aqueous solution. The concentration is more preferably 35 mass% or more and 50 mass% or less. When the above-mentioned concentration is applied, the sol state can be stably maintained. The gelatin aqueous solution (spinning solution) preferably has a viscosity of 500 mPa·s or more and 3000 mPa·s or less. When the viscosity of the gelatin aqueous solution is within the range above, stable spinning can be achieved.

The spinning solution is discharged from a nozzle of a spinning machine, a pressure fluid is supplied from around the nozzle, the pressure fluid is allowed to accompany the discharged gelatin aqueous solution to form fibers, and the obtained gelatin fibers are accumulated on a gelatin film to form a gelatin nonwoven. The nozzle discharge pressure is not particularly limited, but may be, for example, 0.1 MPa or more and 1 MPa or less.

The temperature of the pressure fluid is preferably 20°C or higher and 120°C or lower, and more preferably 80°C or higher and 120°C or lower. Depending on the flow rate of the pressure fluid and the temperature of the ambient atmosphere, if the temperature is within the range above, stable spinning can be achieved. It is preferable to use air as the pressure fluid, and the pressure is preferably 0.1 MPa or more and 1 MPa or less. If the pressure is within the range above, the spinning solution discharged into the air from the nozzle discharge outlet can be blown off to form fibers.

The gelatin nonwoven having a desired average fiber diameter can be obtained by adjusting the nozzle diameter (inner diameter) and the like as appropriate.

It is preferable that the layered composite that includes the gelatin nonwoven and the gelatin film is cross-linked. This makes it possible to enhance the morphological stability and the water resistance. The cross-linking may be chemical cross-linking using a compound such as a cross-linking agent, but it is preferable to perform cross-linking using a cross-linking agent having safety in a living organism, or cross-linking using no cross-linking agents from the viewpoint of safety in a living organism. Examples of the cross-linking using no cross-linking agents include thermal cross-linking, electron beam cross-linking, radiation cross-linking using γ rays or the like, and ultraviolet cross-linking. In the case of electron beam cross-linking, and radiation cross-linking using γ rays or the like, sterilization and cross-linking can also be simultaneously performed. Thermal cross-linking is preferable, and dehydrothermal crosslinking is more preferable, from the viewpoint of easily obtaining a desired cross-linking effect. The dehydrothermal crosslinking may also be performed, for example, at a temperature of 100°C or higher and 160°C or lower for 24 hours or longer and 96 hours or shorter. The dehydrothermal crosslinking may also be performed, for example, under vacuum where the degree of vacuum is 1 kPa or less. The layered composite may be dried before cross-linking is performed. The drying may be air-drying performed at room temperature, or vacuum freeze-drying.

The layered composite in which the gelatin nonwoven and the gelatin film are integrated can be cut into a predetermined shape and size as needed and used as a scaffold. Since gelatin is biocompatible and biodegradable, the layered composite in which the gelatin nonwoven and the gelatin film are integrated is suitable as a medical scaffold and a cell culture scaffold. The scaffold can be sterilized through ethylene oxide gas sterilization, steam sterilization (autoclave), electron beam irradiation, irradiation of radiation such as γ rays, or the like, or disinfected through ethanol treatment or the like. In the case of electron beam irradiation, and irradiation of radiation such as γ rays, sterilization and cross-linking can also be simultaneously performed.

It is preferable to aseptically perform the steps of manufacturing the gelatin film and the layered composite in, for example, a clean bench or a clean room. It is possible to prevent the gelatin film and the layered composite from being contaminated due to the growth of miscellaneous bacteria during the operation. It is preferable that manufacturing instruments to be used are sterilized through, for example, autoclaving, electron beam irradiation, irradiation of radiation such as γ rays, or the like. Also, it is preferable that the gelatin solution is sterilized through, for example, conventionally known filter filtration and is then subjected to the film manufacturing step.

In an example of the present invention, the cross-linked layered composite is shaped into a predetermined shape through, for example, punching, and is then used as a scaffold. Alternatively, after swelling with water, a buffer, or a predetermined liquid culture medium, the layered composite is used as an intended scaffold.

In one or more embodiments of the present invention, it is sufficient that the multicellular construct includes the scaffold and cells, and the cells are present in at least one of a region on the surface of the gelatin nonwoven and a region inside the nonwoven. However, it is preferable that the cells are present in both a region on the surface of the gelatin nonwoven and a region inside the nonwoven. A three-dimensionally cultured cell population in which cells are three-dimensionally arranged in this manner can be used as a tissue. In one or more embodiments of the present invention, the multicellular construct can be formed into various shapes such as a sheet-shape and a block shape as needed. Also, a configuration may also be employed in which the multicellular construct has a structure constituted by two or more layers, each of the layers includes the scaffold and cells, and the cells are present in at least one of a region on the surface of the gelatin nonwoven and a region inside the gelatin nonwoven, preferably both of the regions.

In one or more embodiments of the present invention, it is sufficient that the cells are animal cells, and there is no particular limitation on the origin of the cells. The animal cell may be derived human or non-human. Examples of the non-human animal include primates such as a monkey and a chimpanzee, rodents such as a mouse, a rat, and a hamster, and ungulates such as a cattle, a sheep, a goat, and a swine. Also, in the present invention, the cells include individual cells, cell lines, and cells obtained through cell culture such as primary culture. Examples of the cells include, but are not particularly limited to, somatic cells, stem cells, precursor cells, germ cells, and immune cells.

The somatic cells include somatic cells included in a living organism, and cancer cells derived from somatic cells. Examples of the somatic cells included in a living organism include, but are not particularly limited to, fibroblasts, muscle cells, endothelial cells, osteoblasts, bladder cells, lung cells, osteocytes, neurons, hepatocytes, chondrocytes, epithelial cells, and mesothelial cells. Examples of the cancer cells include, but are not particularly limited to, breast cancer cells, renal cancer cells, prostatic cancer cells, lung cancer cells, hepatic cancer cells, cervical cancer cells, esophageal epithelial cancer, pancreatic cancer, large bowel cancer, and bladder cancer.

The stem cells are cells that can differentiate into various types of specialized cells. Examples of the stem cells include, but are not particularly limited to, embryonic stem cells (ES cells), embryonic carcinoma cells (EC), embryonic germ cells (EG), induced pluripotent stem cells (iPS cells), adult stem cells, blastocyst-derived stem cells, genital ridge-derived stem cells, teratoma-derived stem cells, oncostatin independent stem cells (OISC), bone marrow-derived mesenchymal stem cells, fat-derived mesenchymal stem cells, amniotic fluid-derived mesenchymal stem cells, skin-derived mesenchymal stem cells, and periosteal-derived mesenchymal stem cells.

The precursor cells are cells that originate from the stem cells and can differentiate into terminally differentiated cells included in a living organism.

Examples of the germ cells include spermatozoa, spermatids, ova, and egg cells.

Examples of the immune cells include, but are not particularly limited to, macrophages, lymphocytes, and dendritic cells.

The above-described cells may be used alone or in combination of two or more in accordance with the purposes and the like. For example, the cells preferably include one or more types of cells selected from the group consisting of stem cells; cancer cells; cardiomyocytes, neurons, hepatocytes, fibroblasts, endothelial cells, and epithelial cells that are derived from stem cells; and cardiomyocytes, neurons, hepatocytes, fibroblasts, endothelial cells, and epithelial cells that are derived from a living organism.

In one or more embodiments of the present invention, the multicellular construct can be produced by arranging the scaffold in a swollen state inside the culture vessel whose inner surface is in the dry state such that the gelatin film of the scaffold is in contact with the inner bottom surface of the culture vessel, dripping a cell suspension onto the gelatin nonwoven of the scaffold to seed cells, and then culturing the cells, but there is no particular limitation to this process. This makes it possible to suppress desorption of the cells from the scaffold, enhance the seeding efficiency, and favorably culture the cells at high density from the beginning of the culture. Specifically, it is possible to arrange the scaffold while holding an end portion of the scaffold with tweezers.

One or more embodiments of the present invention can provide a cell seeding method in which cells are seeded on a scaffold, and the method includes: preparing a scaffold constituted by a layered composite that includes a gelatin nonwoven containing gelatin as a main component and a gelatin film layered on one surface of the gelatin nonwoven and containing gelatin as a main component, and a culture vessel whose inner surface has not undergone a hydrophilization treatment; arranging the scaffold in a swollen state inside the culture vessel whose inner surface is in a dry state such that the gelatin film of the scaffold is in contact with the inner bottom surface of the culture vessel; and dripping a cell suspension on the gelatin nonwoven of the scaffold. With this cell seeding method, it is possible to suppress desorption of the cells from the scaffold and enhance the seeding efficiency. Also, one or more embodiments of the present invention can provide a cell culturing method in which the cell suspension is dripped on the gelatin nonwoven of the scaffold and left to stand for a predetermined period of time in the above-mentioned cell seeding method, and then a liquid culture medium is added thereto to culture the cells. With this cell culturing method, it is possible to culture the cells at high density from the beginning of the culture.

In one or more embodiments of the present invention, there is no particular limitation on the culture vessel as long as the inner surface thereof that comes into contact with a cell culture scaffold and a liquid culture medium has not undergone a hydrophilization treatment, and, for example, a dish, a plate, a flask, and the like can be used as appropriate. Specifically, a non-treated (non-cell-adhesive) culture dish that has not undergone a process for enhancing cell-adhesiveness (adherence) can be used, and a culture dish for suspension culture can be favorably used. For example, commercially available products such as "IWAKI Multiwell Plate, Non-treated surface for suspension cell culture 6 Well" and "Corning Coaster ® 6-well Clear Not Treated Multiple Well Plate" can be used.

A liquid culture medium in which cells are suspended can be used as the cell suspension. The liquid amount (dripping amount) of the cell suspension per unit surface area of the scaffold can be determined as appropriate in accordance with the thickness and basis weight of the scaffold, the concentration of the cell suspension, and the like, and is not particularly limited. This amount is preferably 0.1 $\mu$L/mm$^2$ or more and 0.6 $\mu$L/mm$^2$ or less, more preferably 0.2 $\mu$L/mm$^2$ or more and 0.5 $\mu$L/mm$^2$ or less, and even more preferably 0.3 $\mu$L/mm$^2$ or more and 0.4 $\mu$L/mm$^2$ or less. If the amount is within the range above, the cell suspension is likely to be uniformly distributed inside the scaffold and to be held inside the scaffold.

The cell seeding amount per unit surface area of the scaffold is not particularly limited, and can be determined as appropriate based on the thickness and basis weight of the scaffold, and the like. For example, this amount is preferably 200 cells/mm$^2$ or more and 20000 cells/mm$^2$ or less, more preferably 2000 cells/mm$^2$ or more and 15000 cells/mm$^2$ or less, and even more preferably 4000 cells/mm$^2$ or more and 12000 cells/mm$^2$ or less, from the viewpoint of high-density seeding.

The liquid culture medium is not particularly limited, and a liquid culture medium containing a component that is necessary for survival and growth of cells can be used as appropriate. The culture medium may also contain serum, an antibiotic, a growth factor, and the like. As the serum, for example, bovine serum, fetal bovine serum, horse serum, human serum, and the like can be used as appropriate. As the antibiotic, penicillin, streptomycin, gentamicin, amphotericin, ampicillin, minomycin, kanamycin, and the like can be used as appropriate. As the growth factor, a cell growth factor, a differentiation inducing factor, a cell adhesion factor, and the like can be used as appropriate.

In one or more embodiments of the present invention, after the cell suspension has been dripped on the gelatin nonwoven of the scaffold and then allowed to stand for a predetermined period of time (e.g., 3 to 4 hours) to perform preculture for allowing the cells to adhere to the gelatin nonwoven (scaffold), the liquid culture medium can be added thereto to start cell culturing.

The culture may be performed at a temperature of, for example, 27°C or higher and 40°C or lower, or 31°C or higher and 37°C or lower. The concentration of carbon dioxide may be within a range of 2% or more and 10% or less.

It is sufficient that the culture time is determined as appropriate in accordance with the type of cells, the number of cells, and the like. For example, the culture may be continued for 2 to 21 days, or 3 to 14 days, or 4 to 10 days. The culture medium may be replaced every 2 to 4 days.

For example, a process may be employed in which, 3 to 4 hours after the cell seeding, it is confirmed that the cells have adhered to the scaffold, and then the liquid culture medium is added to the culture vessel to start stationary culturing in an incubator under predetermined conditions (e.g., 37°C and 5% $CO_2$). The liquid culture medium may be replaced every 2 to 4 days. Alternatively, a process may be employed in which, after the cell seeding, the liquid culture medium is added to the culture vessel, and then spinner culture is performed on a magnetic stirrer placed in an incubator at 37°C and 5% $CO_2$ with the liquid culture medium being stirred and circulated. The medium may be replaced every 3 to 4 days as follows: a half of the liquid culture medium is removed, and then an equal amount of new liquid culture medium is added. Alternatively, a process may be employed in which, after the cell seeding, the liquid culture medium is added to the culture vessel, and then shaking culture is performed in an incubator at 37°C and 5% $CO_2$. The medium may be replaced every 3 to 4 days as follows: a half of the liquid culture medium is removed, and then an equal amount of new liquid culture medium is added. The scaffold according to the present invention becomes transparent when getting wet with water, thus making it possible to observe even the inside of the scaffold in the culture liquid under an inverted microscope.

Seeding cells on the scaffold as described above makes it possible to enhance the seeding efficiency and perform high-density seeding, and performing cell culture after the seeding makes it possible to perform high-density culture.

One or more embodiments of the present invention can provide a kit for producing a multicellular construct, and the kit includes: the above-described scaffold constituted by a layered composite that includes a gelatin nonwoven containing gelatin as a main component and a gelatin film layered on one surface of the gelatin nonwoven and containing gelatin as a main component; and a culture vessel whose inner surface has not undergone a hydrophilization treatment, wherein, during production of the multicellular construct, the scaffold in a swollen state is arranged inside the culture vessel whose inner surface is in a dry state such that the gelatin film of the scaffold is in contact with the inner bottom surface of the culture vessel, and a cell suspension is dripped on the gelatin nonwoven of the scaffold. The kit for producing a multicellular construct as described above can also be used as a cell seeding kit or a cell culture kit.

In one or more embodiments of the present invention, if the above-mentioned cardiomyocytes are cardiomyocytes that have differentiated from stem cells or directly reprogrammed somatic cells, the scaffold can promote the maturation of cardiomyocytes, and thus mature cardiomyocytes and a multicellular construct of cardiomyocytes can be obtained. In one or more preferable embodiments of the present invention, if the cardiomyocytes are present on the gelatin fibers included in the scaffold (gelatin nonwoven), the cells attaching to the gelatin fibers will be oriented in the longitudinal direction of the fibers because a region in which the cells extend is wider in the longitudinal direction of the fibers than in the diametrical direction of the fibers. In addition, the gelatin nonwoven is flexible and has excellent deformation recovery properties. Accordingly, mechanical stimulus is applied to the cardiomyocytes due to the gelatin fibers contracting and relaxing together with the cardiomyocytes, thus making it possible to promote maturation of the cardiomyocytes.

Cardiomyocytes that have differentiated from stem cells or directly reprogrammed somatic cells are more immature than human primary cardiomyocytes and have insufficient functions in some cases. If immature cardiomyocytes are used to detect a side effect of a compound, ion channels associated with the prediction about induction of arrhythmia, the development of a sarcomere structure associated with cardiac contraction dysfunction, and the contractile force enhancing effect (positive inotropic effect) caused by addition of a drug are not observed in some cases.

Applying external stimulus such as stretch stimulus or electric stimulus to cardiomyocytes is known as a method for promoting maturation of cardiomyocytes, but it is necessary to use a special culture dish in this method. In one or more embodiments of the present invention, even if the cardiomyocytes are cardiomyocytes that have differentiated from stem cells or directly reprogrammed somatic cells, a multicellular construct of mature cardiomyocytes can be obtained without using a special culture dish.

Examples of the features of mature cardiomyocytes include the structure, contractibility, electrophysiology, calcium handling, and metabolism of cardiomyocytes. Examples of the structural features of mature cardiomyocytes include an increased size, a vertically long cell shape, an oriented sarcomere structure, and a sarcomere length of 1.8 $\mu$m to 2.2 $\mu$m. Examples of the contractibility features of mature cardiomyocytes include increased contractile force, increased $\alpha$-MHC/$\beta$-MHC, a change of titin N2A to titin N2B, contractile force increased by $\beta$-adrenergic receptor stimulation, and a protein kinase A-dependent increase in contractile force. Examples of the electrophysiological features of mature cardiomyocytes include an increased excitation conduction velocity, a reduced resting membrane potential, and a prolonged action potential duration. Examples of the calcium handling features of mature cardiomyocytes include an increased amount of calcium stored in sarcoplasmic reticula, an increased calcium cycle caused by adrenaline stimulation, and the development of the T-tubule. Examples of the metabolic features of mature cardiomyocytes include use of fatty acids and the increased number of mitochondria. The above-mentioned features of cardiomyocytes can be confirmed through imaging measurements, contractile force measurements, a contractile speed, pharmacological evaluations, gene analyses, and the like.

Examples of genes expressed in mature cardiomyocytes include: SCN5A, KCNJ2, GJA1, and HCN4, which are associated with electrophysiology; ATP2A2, CACNA1C, RYR2, SLC8A1, PLN, BIN1, and JPH2, which are associated with calcium handling; MYH7, MYH6, MYL2, TNNI3, PLN, SERCA2A, which are associated with contraction; and CPT1B, PGC1A, and TFAM, which are associated with metabolism.

In one or more embodiments of the present invention, the multicellular construct can be favorably used in cell transplantation therapies, compound safety evaluations, compound toxicity evaluations, medical devices, disease models, drug discovery research using disease models, and the like.

In one or more embodiments of the present invention, the multicellular construct can be used in, for example, a method for evaluating a characteristic of a compound. Specifically, the characteristics of a compound can be evaluated by delivering the compound into contact with the multicellular construct and then determining whether or not the physiological properties of the multicellular construct are changed due to the contact with the compound. The characteristics of the compound may include, for example, metabolism, a pharmacological effect, a toxic effect, and the like, but are not limited thereto. More specifically, a process may be employed in which, after a multicellular construct has been obtained by seeding cells on the scaffold and culturing the cells for a predetermined period of time as described above, a target compound is added to the liquid culture medium used for the cell culture, and it is observed and evaluated whether or not the physiological properties of the multicellular construct are changed after the addition of the compound.

When the cells are cardiomyocytes, the multicellular construct can be used to evaluate the influences of the target compound on a risk of arrhythmia induction, cardiac contraction dysfunction, and cardio-toxicity by evaluating electrophysiological properties, cardiac contraction, and the like. In one or more preferable embodiments of the present invention, a multicellular construct in which cardiomyocytes are present on the surface of the scaffold and inside the scaffold and adhere to the scaffold (specifically, adhere to the gelatin fibers and the surface on the gelatin nonwoven side of the gelatin film), namely a three-dimensionally cultured multicellular construct in which cardiomyocytes are three-dimensionally arranged, is likely to follow the movement of the cardiomyocytes as a whole when the cardiomyocytes contract, and can be used to effectively evaluate the functionality of the cardiomyocytes such as the heartbeat, the action potential, and the contractile behavior. Specifically, the action potential of the multicellular construct can be evaluated using intracellular calcium ion imaging, cell membrane potential imaging, a microelectrode array, and the like. The contractile behavior can be evaluated by observing the movement of the scaffold and the fibers included in the scaffold, and a bright field, calcium ion imaging, membrane potential imaging, and the like can be used freely.

Examples

Hereinafter, a more specific description will be given by way of examples. Note that the present invention is not limited to the following examples.

The measurement and evaluation methods are as follows.

Average Fiber Diameter

A swollen scaffold was observed using a confocal imaging system (CellVoyager CQ1, Yokogawa Electric Corp.), and the average diameter of swollen fibers was measured using randomly selected fifty fibers.

Thickness

A cross section of a layered composite (scaffold) was observed using a scanning electron microscope (Flex SEM 1000, manufactured by Hitachi High-Technologies Corporation; 100- and 500-fold magnification), and the thickness of the gelatin film layer, the thickness of the gelatin nonwoven, and the thickness of the layered composite were measured at each of ten randomly selected points in the obtained scanning electron micrograph. Then, the average values thereof were calculated.

The thickness of the swollen layered composite (scaffold) was measured using a digital caliper manufactured by Mitutoyo Corporation.

Basis Weight (Mass per Unit Area)

The basis weight of a gelatin nonwoven was measured in conformity with JIS L 1913.

Apparent Density

The density of a gelatin nonwoven was calculated based on the thickness and the basis weight of the nonwoven.

Pore Diameter

The pore diameter of a gelatin nonwoven was calculated using Computational Expression 1 below based on the Wrotnowski's hypothesis.
[Mathematical Formula 2]

Computational Expression 1

$$D = \frac{42.5}{cos45 \sqrt{\dfrac{W}{F}}} - f$$

$$f = 11.9 \times \sqrt{\frac{F}{\rho}}$$

D: Pore diameter ($\mu$m)
W: Apparent density of nonwoven (g/cm$^3$)
F: Fineness (d)
$f$: Fiber diameter ($\mu$m)
$\rho$: Fiber density (g/cm$^3$)

Example 1

Production of Layered Composite (Scaffold)

Gelatin manufactured by Nitta Gelatin Inc. (jelly strength: 262 g; raw material: alkali-treated cattle bones) was mixed with water such that the mass ratio of the gelatin to water was 95: 5 (the gelatin concentration was 5 mass%), and the gelatin was dissolved at 60°C. This gelatin aqueous solution was applied onto a polytetrafluoroethylene film (film thickness: 50 $\mu$m) using a bar coater No. 20 manufactured by TP Giken Co., Ltd., and a gelatin film was obtained by air-drying the polytetrafluoroethylene film at room temperature overnight.

Next, gelatin manufactured by Nitta Gelatin Inc. (jelly strength: 262 g; raw material: alkali-treated cattle bones) was mixed with water such that the mass ratio of the gelatin to water was 3:5 (the gelatin concentration was 37.5 mass%), and the gelatin was dissolved at 60°C. The viscosity at 60°C was 960 to 970 mPa·s. This gelatin aqueous solution was used as a spinning solution, and the manufacturing apparatus shown in FIG. 23 was used to produce a layered composite by accumulating gelatin fibers to form nonwoven on a gelatin film arranged on a winding roll. The temperature of the spinning solution was 60°C, the nozzle diameter (inner diameter) was 250 $\mu$m, the discharge pressure was 0.2 MPa, the nozzle height was 5 mm, the air pressure was 0.375 MPa, the air temperature was 100°C, the distance between the fluid spout and the nozzle discharge outlet was 5 mm, and the collection distance was 50 cm. The layered composite was air-dried at room temperature overnight, and was then subjected to dehydrothermal crosslinking. The cross-linking was performed at 140°C for 48 hours.

A cylindrical layered composite having a diameter of 6 mm was produced by punching the obtained layered composite and was used as a scaffold.

Production of Multicellular Construct

(1) The layered composite (scaffold) obtained as mentioned above was sterilized using ethylene oxide gas, and was then left to stand in Dulbecco's Phosphate Buffered Saline (D-PBS(-)),(Nacalai Tesque Inc.) for 10 minutes and thus swollen. The swollen layered composite had a diameter of 8 mm.

(2) Excess liquid was removed from the swollen layered composite using a pipette, and then the layered composite was placed in one well of non-treated (non-cell-adhesive) culture plate (Multiwell Plate, Non-treated surface for suspension cell culture 6 Well, IWAKI) such that the gelatin film was in contact with the inner bottom surface of the well while an end portion of the layered composite was held with tweezers.

(3) 20 pL of a cell suspension obtained by suspending human embryonic kidney cells, HEK293 cells, in a liquid culture medium (Dulbecco's Modified Eagle's Medium (Sigma) supplemented with 10% fetal bovine serum and 1% penicillin-streptomycin) at a concentration of $2\times10^7$ cells/mL was dripped on the surface of the gelatin nonwoven of the layered composite. Static culture was performed in an incubator at 37°C and 5% $CO_2$ for 3 hours to allow the cells to adhere the layered composite. The cell seeding amount per unit area of the scaffold was 6250 cells/mm$^2$.

(4) 3 mL of the above-mentioned liquid culture medium was added, and then static culture was performed in an incubator at 37°C and 5% $CO_2$ for 72 hours.

Example 2

Production of Layered Composite (Scaffold)

A layered composite was produced in the same manner as in Example 1, except that the nozzle diameter (inner diameter) was 150 μm, the nozzle discharge pressure was 0.275 MPa, the nozzle height was 5 mm, the air pressure was 0.375 MPa, the air temperature was 100°C, the distance between the fluid spout and the nozzle discharge outlet was 5 mm, and the collection distance was 50 cm.

A cylindrical layered composite having a diameter of 6 mm was produced by punching the obtained layered composite and was used as a scaffold.

Production of Multicellular Construct

A multicellular construct was produced in the same manner as in Example 1, except that the layered composite (scaffold) obtained as mentioned above was used. The swollen layered composite had a diameter of 8 mm.

Example 3

Production of Layered Composite (Scaffold)

A cylindrical layered composite having a diameter of 6 mm was produced by punching a layered composite obtained in the same manner as in Example 1 and was used as a scaffold. The swollen layered composite had a diameter of 8 mm.

The scaffold was immersed, for 2 hours, in a fibronectin-containing phosphate buffer obtained by adding 1mg/ml fibronectin solution (Sigma) to Dulbecco's phosphate-buffered saline (D-PBS(-)), (Nacalai Tesque Inc.) at a volume ratio of 1/100, and then the fibronectin solution was removed by aspiration.

Production of Multicellular Construct

A multicellular construct was produced in the same manner as in Example 1, except that the fibronectin-treated scaffold and a cell suspension obtained by suspending human iPS cells derived cardiomyocytes (iCell $^®$ Cardiomyocytes$^2$, FUJIFILM Wako Pure Chemical Corp.) in a liquid culture medium (iCell $^®$ Cardiomyocyte Plating Medium, FUJIFILM Wako Pure Chemical Corp.) at a concentration of $1\times10^7$ cells/mL were used, and a liquid culture medium (iCell $^®$ Cardiomyocyte Maintenance Medium, FUJIFILM Wako Pure Chemical Corp.) was added 4 hours after the start of the culture. The cell seeding amount per unit area of the scaffold was 3125 cells/mm$^2$.

Example 4

Production of Layered Composite (Scaffold)

A cylindrical layered composite having a diameter of 6 mm was produced by punching a layered composite obtained in the same manner as in Example 2 and was used as a scaffold. The swollen layered composite had a diameter of 8 mm.

The scaffold was immersed, for 2 hours, in a fibronectin-containing phosphate buffer obtained by adding 1mg/ml fibronectin solution (Sigma) to Dulbecco's phosphate-buffered saline (D-PBS(-)), (Nacalai Tesque Inc.) at a volume ratio of 1/100, and then the fibronectin-containing liquid culture medium was removed by aspiration.

Production of Multicellular Construct

A multicellular construct was produced in the same manner as in Example 2, except that the fibronectin-treated scaffold and a cell suspension obtained by suspending human iPS cell derived cardiomyocyte (iCell [®] Cardiomyocytes[2], FUJIFILM Wako Pure Chemical Corp.) in a liquid culture medium (iCell [®] Cardiomyocyte Plating Medium, FUJIFILM Wako Pure Chemical Corp.) at a concentration of $1\times10^7$ cells/mL were used, and a liquid culture medium (iCell [®] Cardiomyocyte Maintenance Medium, FUJIFILM Wako Pure Chemical Corp.) was added 4 hours after the start of the culture. The cell seeding amount per unit area of the scaffold was 3125 cells/mm[2].

Comparative Example 1

Cell seeding and cell culture were performed in the same manner as in Example 1, except that "IWAKI Multiwell Plate, TC-treated surface for adhesive cell culture 6 Well" was used as a culture vessel.

Comparative Example 2

Cell seeding and cell culture were performed in the same manner as in Example 3, except that "IWAKI Multiwell Plate, TC-treated surface for adhesive cell culture 6 Well" was used as a culture vessel.

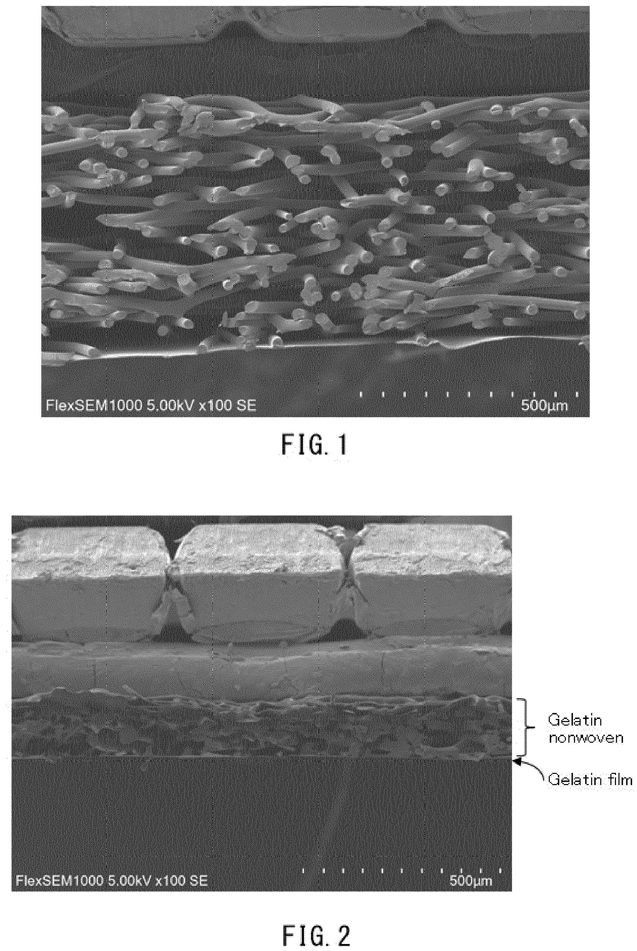

Table 1 below shows the results of various measurements and evaluations performed on the scaffolds used in the examples. FIGS. 1 and 2 show photographs of the cross sections of the scaffolds used in Examples 1 and 2, respectively. As is clear from FIGS. 1 and 2, the gelatin fibers included in the gelatin nonwoven were welded to one another at some or all of fiber cross-points, and the gelatin film was welded to some of the gelatin fibers included in the gelatin nonwoven.

Table 1

|  |  | Ex. 1 | Ex. 2 |
|---|---|---|---|
| Scaffold (swollen) | Thickness ($\mu$m) | 800 | 500 |
| Scaffold | Thickness ($\mu$m) | 550.18 | 135.75 |
| Gelatin film | Thickness (Tf) ($\mu$m) | 1.68 | 1.25 |
| Gelatin nonwoven | Thickness (Tn) ($\mu$m) | 548.5 | 134.5 |
|  | Basis weight (g/mm$^2$) | 150 | 50 |
|  | Average fiber diameter after swelling ($\mu$m) | 47 | 20 |
|  | Pore diameter ($\mu$m) | 119.6 to 157.1 | 50.9 to 66.8 |

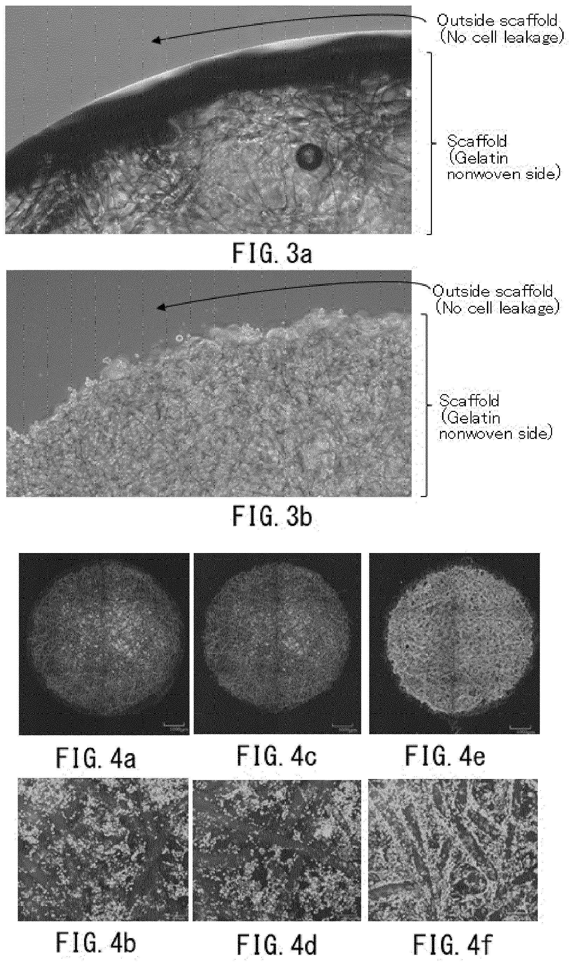

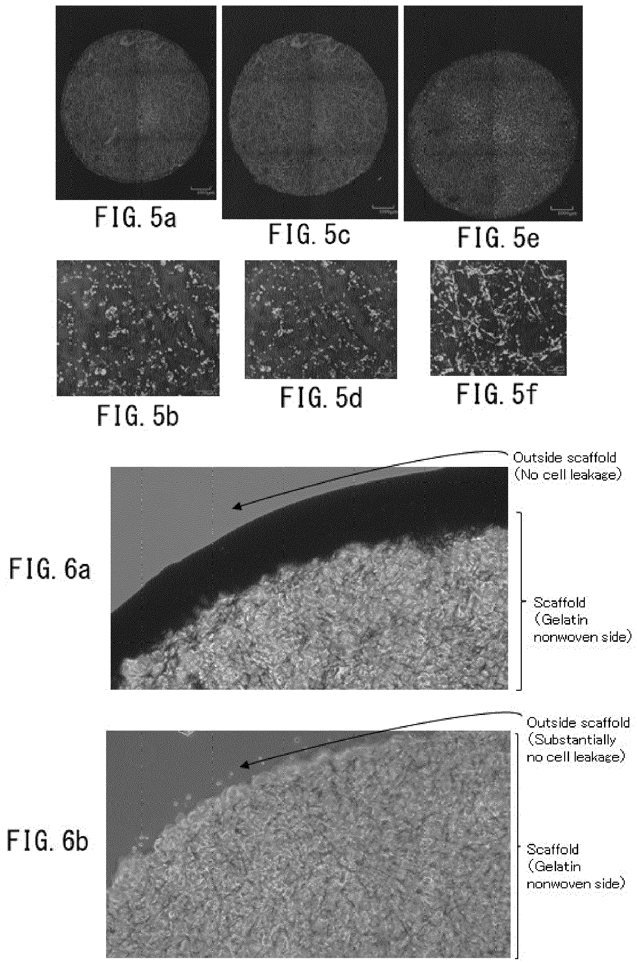

In Examples 1 and 2 and Comparative Example 1, the scaffold was observed as described below just after the cells had been seeded (also referred to as "after the seeding" hereinafter), after the scaffold had been cultured for 3 hours and the liquid culture medium had been added thereto (also referred to as "after the 3-hour culture" hereinafter), and after the scaffold had been cultured for 72 hours. FIGS. 3 to 5 and 9, and Table 2 show the results. In Examples 3 and 4 and Comparative Example 2, the scaffold was observed as described below just after the cells had been seeded (also referred to as "after the seeding" hereinafter), after the scaffold had been cultured for 4 hours and the liquid culture medium had been added thereto (also referred to as "after the 4-hour culture" hereinafter), and after the scaffold had been cultured for 72 hours. FIGS. 6 to 8 and 10, and Table 3 show the results.

(1) Whether or Not Cells Desorbed

An end portion of the scaffold was observed under an optical microscope (Axio Vert.A1, Zeiss) at 4-fold magnification after the seeding and after the 3-hour culture (Examples 1 and 2) or 4-hour culture (Examples 3 and 4).

(2) Fluorescent Observation of Viable Cells

A DMSO solution of a nuclear staining fluorescence reagent, 1 mg/mL bisbenzimide H33342 fluorochrome trihydrochloride (Nacalai Tesque Inc.) was added to the liquid culture medium at a volume ratio (v/v) of 1/100, and the culturing was performed at 37°C and 5% $CO_2$ for 30 minutes. Then, the scaffold was washed using Dulbecco's phosphate-buffered saline (D-PBS(-)), (Nacalai Tesque Inc.), and the liquid culture medium was added again.

Thereafter, fluorescent observation of viable cells was conducted using a confocal imaging system (CellVoyager CQ1, Yokogawa Electric Corp.). In order to observe both the front and back of the layered construct, the layered construct was inverted while an end portion thereof was held with tweezers, and then the layered construct was observed. Observation images were taken at 10-$\mu$m intervals in the thickness direction, and 50 images were superimposed using maximum intensity projection (MIP). The image of the entire scaffold was obtained by linking the 4-fold observation images. The magnified image was a 20-fold magnified observation image. Portions that look white in the images correspond to cell nuclei.

(3) Calculation of Number of Viable Cells and Ratio of Viable Cells

After the scaffold had been washed using Dulbecco's phosphate-buffered saline (D-PBS(-)), (Nacalai Tesque Inc.) a day after the seeding and then moved to a 15-mL centrifugal tube, 1 mL of a 2.5 g/L trypsin / 1 mmol/L EDTA solution (Nacalai Tesque Inc.) was dripped thereon. After culture had been performed at 37°C and 5% $CO_2$ for 25 minutes, and dissolution of the scaffold had been visually observed, dead cells were stained using a 0.5% trypan blue staining solution (Nacalai Tesque Inc.), and the number of viable cells and the ratio of the viable cells were calculated using automatic cell counter (TC20 Automated Cell Counter, Bio-Rad Laboratories Inc.).

(4) Cell Distribution in Scaffold

A DMSO solution of a nuclear staining fluorescence reagent, 1mg/mL bisbenzimide H33342 fluorochrome trihydrochloride(Nacalai Tesque Inc.) was added to the liquid culture medium at a volume ratio (v/v) of 1/100, and the scaffold after the 72-hour culture was cultured at 37°C and 5% $CO_2$ for 30 minutes. Then, the scaffold was washed using Dulbecco's phosphate-buffered saline (D-PBS(-)), (Nacalai Tesque Inc.), and the liquid culture medium was added again.

Thereafter, fluorescent observation of viable cells was conducted using a confocal imaging system (CellVoyager CQ1, Yokogawa Electric Corp.). In order to observe both the front and back of the layered construct, the layered construct was inverted while an end portion thereof was held with tweezers, and then the layered construct was observed. The observation was performed at 10-$\mu$m intervals in the thickness direction, and positions 0 $\mu$m (i.e., on the surface), 100 $\mu$m, and 200 $\mu$m away from the surface of the gelatin nonwoven and the surface of the gelatin film in the thickness direction were observed to check the distribution of cells in the depth direction.

FIG. 3 shows images of an end portion of the scaffold observed under an optical microscope (4-fold magnification) in Example 1. FIG. 3(a) shows the result obtained after the cell seeding, and FIG. 3(b) shows the result obtained when the liquid culture medium was added after the 3-hour culture.

FIG. 4 shows fluorescence images of the entire scaffold when observing viable cells (HEK293 cells) in the scaffold. FIG. 4(a) is an image of the entire scaffold after the cell seeding, FIG. 4(b) is an enlarged image of the central portion of the scaffold shown in FIG. 4(a), FIG. 4(c) is an image of the entire scaffold (multicellular construct) taken when the liquid culture medium was added after the 3-hour culture, FIG. 4(d) is an enlarged image of the central portion of the scaffold shown in FIG. 4(c), FIG. 4(e) is an image of the entire scaffold (multicellular construct) taken after the 72-hour culture, and FIG. 4(f) is an enlarged image of the central portion of the scaffold shown in FIG. 4(e). The scale bars in FIGS. 4(a), 4(c), and 4(e) indicate 1000 $\mu$m, and the scale bars in FIGS. 4(b), 4(d), and 4(f) indicate 100 $\mu$m.

FIG. 5 shows fluorescence images of the entire scaffold when observing viable cells (HEK293 cells) in the scaffold in Example 2. FIG. 5(a) is an image of the entire scaffold after the cell seeding, FIG. 5(b) is an enlarged image of the central portion of the scaffold shown in FIG. 5(a), FIG. 5(c) is an image of the entire scaffold (multicellular construct) taken when a liquid culture medium was added after the 3-hour culture, FIG. 5(d) is an enlarged image of the central portion of the scaffold shown in FIG. 5(c), FIG. 5(e) is an image of the entire scaffold (multicellular construct) taken after the 72-hour culture, and FIG. 5(f) is an enlarged image of the central portion of the scaffold shown in FIG. 5(e). The scale bars in FIGS. 5(a), 5(c), and 5(e) indicate 1000 $\mu$m, and the scale bars in FIGS. 5(b), 5(d), and 5(f) indicate 100 $\mu$m.

FIG. 6 shows images of an end portion of the scaffold observed under an optical microscope (4-fold magnification) in Example 3. FIG. 6(a) shows the result obtained after the cell seeding and FIG. 6(b) shows the result obtained when the liquid culture medium was added after the 3-hour culture.

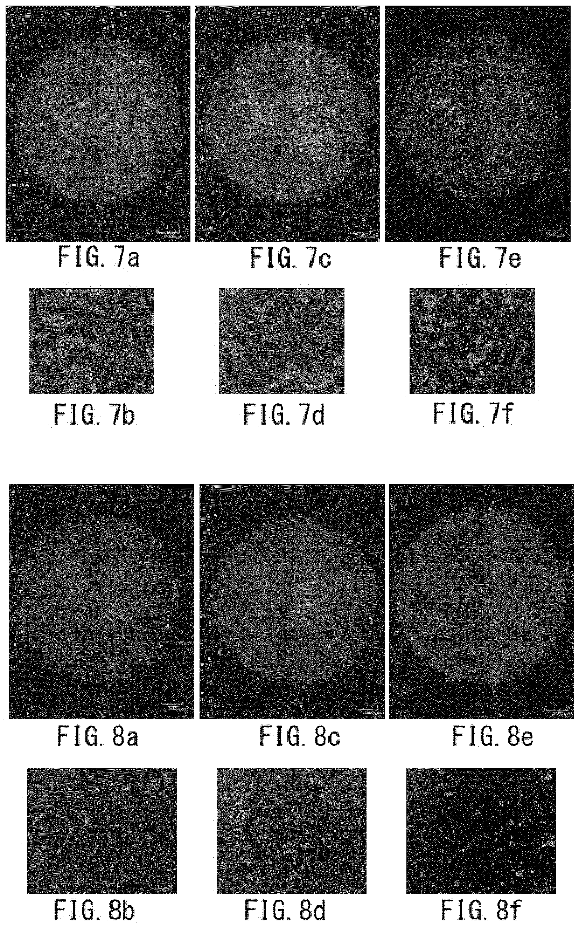

FIG. 7 shows fluorescence images of viable cells (cardiomyocytes) in the scaffold observed in Example 3. FIG. 7(a) is an image of the entire scaffold after the cell seeding, FIG. 7(b) is an enlarged image of the central portion of the scaffold shown in FIG. 7(a), FIG. 7(c) is an image of the entire scaffold (multicellular construct) taken when a liquid culture medium was added after the 3-hour culture, FIG. 7(d) is an enlarged image of the central portion of the scaffold shown in FIG. 7(c), FIG. 7(e) is an image of the entire scaffold (multicellular construct) taken after the 72-hour culture, and FIG. 7(f) is an enlarged image of the central portion of the scaffold shown in FIG. 7(e). The scale bars in FIGS. 7(a), 7(c), and 7(e) indicate 1000 $\mu$m, and the scale bars in FIGS. 7(b), 7(d), and 7(f) indicate 100 $\mu$m.

FIG. 8 shows fluorescence images of viable cells (cardiomyocytes) in a scaffold observed in Example 4. FIG. 8(a) is an image of the entire scaffold after the cell seeding, FIG. 8(b) is an enlarged image of the central portion of the scaffold shown in FIG. 8(a), FIG. 8(c) is an image of the entire scaffold (multicellular construct) taken when a liquid culture medium was added after the 3-hour culture, FIG. 8(d) is an enlarged image of the central portion of the scaffold shown in FIG. 8(c), FIG. 8(e) is an image of the entire scaffold (multicellular construct) taken after the 72-hour culture, and FIG. 8(f) is an enlarged image of the central portion of the scaffold shown in FIG. 8(e). The scale bars in FIGS. 8(a), 8(c), and 8(e) indicate 1000 $\mu$m, and the scale bars in FIGS. 8(b), 8(d), and 8(f) indicate 100 $\mu$m.

As is clear from FIGS. 3 to 8, in Examples 1 to 4, substantially no cells desorbed from the scaffold after the cell seeding, and the same applies at the time of the addition of the liquid culture medium after the 3-hour culture (Examples 1 and 2) or 4-hour culture (Examples 3 and 4), and after the 72-hour culture. That is, the seeding efficiency was high, and high-density culturing was possible from the beginning.

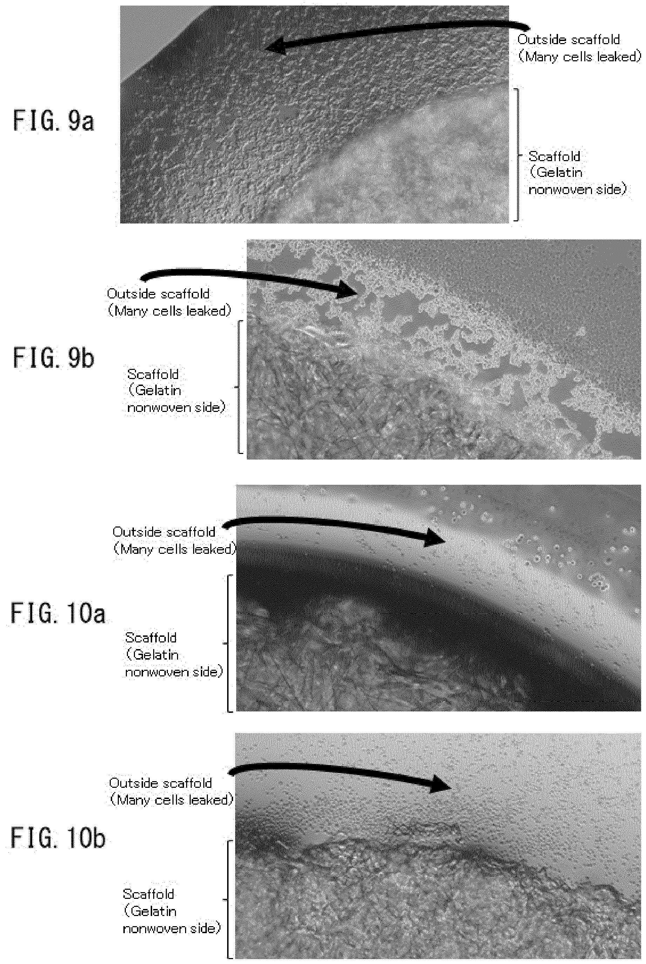

FIG. 9 shows results of the observation performed under an optical microscope (4-fold magnification) to check whether or not cells desorbed from an end portion of the scaffold in Comparative Example 1. FIG. 9(a) shows the result obtained after the cell seeding and FIG. 9(b) shows the result obtained when the liquid culture medium was added after the 3-hour culture. As is clear from FIG. 9, in Comparative Example 1, many cells desorbed from the scaffold just after the cell seeding, and the seeding efficiency was low.

Table 2

|                         | Ex. 1   | Ex. 2    | Comp. Ex. 1 |
|-------------------------|---------|----------|-------------|
| Viable cell number      | 39133.3 | 395333.3 | 250000.0    |
| Seeding efficiency (%)  | 97.8    | 98.8     | 62.5        |
| Viable cell ratio (%)   | 96.5    | 94.5     | 98          |

As is clear from the data shown in Table 2, the seeding efficiency in Comparative Example 1 was 62.5%, whereas the seeding efficiency in Examples 1 and 2 was 97.8% or more, which means that the seeding efficiency significantly improved.

FIG. 10 shows results of the observation performed under an optical microscope (4-fold magnification) to check whether or not cells desorbed from an end portion of the scaffold in Comparative Example 2. FIG. 10(a) shows the result obtained after the cell seeding and FIG. 10(b) shows the result obtained when the liquid culture medium was added after the 4-hour culture. As is clear from FIG. 10, in Comparative Example 2, many cells desorbed from the scaffold after the cell seeding, and the seeding efficiency was low.

Table 3

|                         | Ex. 3   | Ex. 4   | Comp. Ex. 2 |
|-------------------------|---------|---------|-------------|
| Viable cell number      | 63466.7 | 95500.0 | 20950.0     |
| Seeding efficiency (%)  | 31.7    | 47.8    | 10.5        |
| Viable cell ratio (%)   | 78.3    | 80.0    | 93.0        |

As is clear from the data shown in Table 3, the seeding efficiency in Comparative Example 2 was 10.5%, whereas the seeding efficiency in Example 3 was 31.7 % and the seeding efficiency in Example 4 was 47.8%, which means that the seeding efficiency significantly improved. The seeding efficiency of iPS-derived cardiomyocytes is low, and even when they are cultured on a cell-adhesive culture dish, the seeding efficiency is about 40 to 50%. Therefore, the seeding efficiency of the scaffold constituted by the gelatin nonwoven and the gelatin film is excellent.

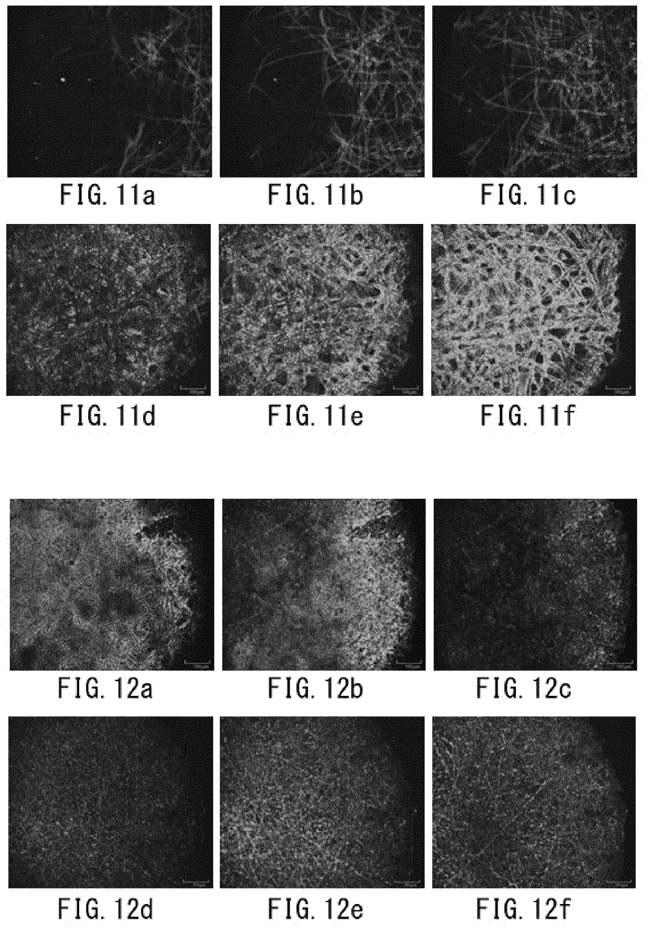

FIG. 11 shows the results of the observation of viable cell distribution in the scaffold after the 72-hour culture in Example 1. FIG. 11(a) shows the result from the observation of the surface on a gelatin nonwoven side, FIG. 11(b) shows the result from a portion that is 100 μm away from the surface of the gelatin nonwoven in the thickness direction, FIG. 11(c) shows the result from a portion that is 200 μm away from the surface of the gelatin nonwoven in the thickness direction, FIG. 11(d) shows the result from a portion that is 200 μm away from the surface of a gelatin film in the thickness direction, FIG. 11(e) shows the result from a portion that is 100 μm away from the surface of a gelatin film in the thickness direction, and FIG. 11(f) shows the result from the observation of the surface on the gelatin film side. The scale bar in FIG. 11 indicates 500 μm.

FIG. 12 shows images illustrating viable cell distribution in the scaffold observed after the 72-hour culture in Example 2. FIG. 12(a) shows the result from the observation of the surface on a gelatin nonwoven side, FIG. 12(b) shows the result from a portion that is 100 μm away from the surface of the gelatin nonwoven in the thickness direction, FIG. 12(c) shows the result from a portion that is 200 μm away from the surface of the gelatin nonwoven in the thickness direction, FIG. 12(d) shows the result from a portion that is 200 μm away from the surface of a gelatin film in the thickness direction, FIG. 12(e) shows the result from a portion that is 100 μm away from the surface of a gelatin film in the thickness direction, and FIG. 12(f) shows the result from the observation of the surface on the gelatin film side. The scale bar in FIG. 12 indicates 500 μm.

As is clear from FIGS. 11 and 12, in the examples, the cells also entered the inside of the scaffold and could be three-dimensionally cultured. In the case of Example 1 in which the gelatin fibers included in the gelatin nonwoven had a large average fiber diameter (i.e., the pore diameter of the gelatin nonwoven was large), the number of cells was small on the gelatin nonwoven side and increased toward the gelatin film in the thickness direction. On the other hand, in the case of Example 2 in which the gelatin fibers included in the gelatin nonwoven had a small average fiber diameter (i.e., the pore diameter of the gelatin nonwoven was small), the number of cells on the gelatin nonwoven side was larger than that on the gelatin film side.

Example 5

Evaluation of Compound Using iPS Cell Derived Cardiomyocytes 1

(1) Evaluation of Synchronous Beating of Cardiomyocytes

Cells were seeded in the same manner as in Example 4, and a liquid culture medium was added 4 hours later to start cell culturing. On the fourth day of the culture, a calcium indicator (EarlyTox Cardiotoxicity Kit, Molecular Devices LLC.) was added to the culture medium at a volume ratio of 5/100 to fluorescently stain calcium ions in the cardiomyocytes.

Videos were captured at 20 FPS using a confocal imaging confocal imaging system (CellVoyager CQ1, Yokogawa Electric Corp.). Fluorescent areas in the obtained videos were recognized using an analysis software Cell-Pathfinder, Yokogawa Electric Corp.), and signal waveforms of calcium ions in the cardiomyocytes were obtained by plotting the fluorescence intensities versus time.

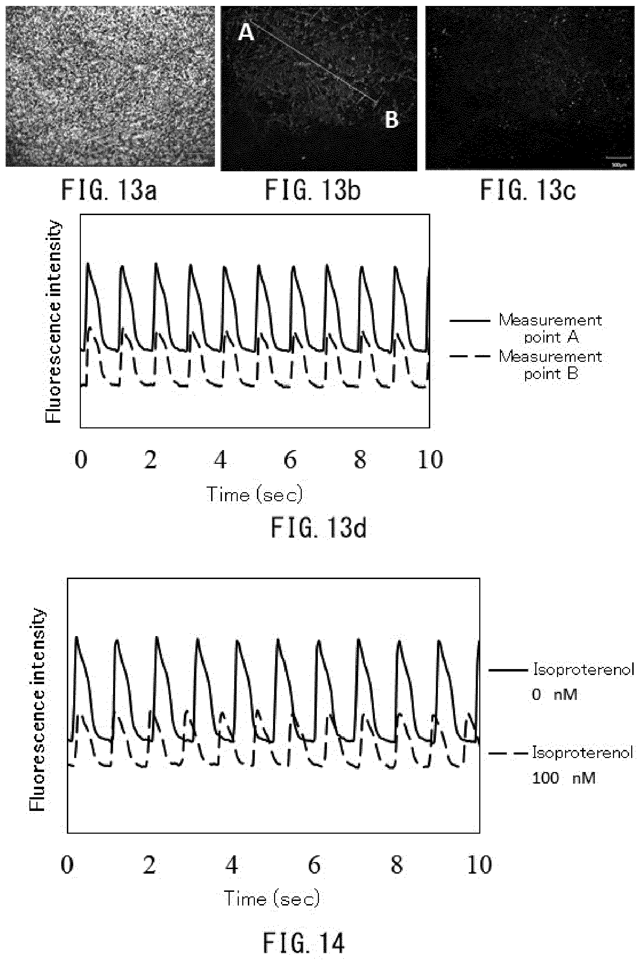

FIG. 13 shows the imaging evaluation results of calcium ions in the cells of the multicellular construct after the calcium indicator had been added on the fourth day of the culture. FIG. 13(a) is a bright field image, FIG. 13(b) is a fluorescence image of intracellular calcium ions observed 120 minutes after the addition of the calcium indicator, FIG. 13(c) is a fluorescence image of intracellular calcium ions observed 0.7 seconds after the image shown in FIG. 13(b) had been taken, and FIG. 13(d) is a graph obtained by plotting the fluorescence intensities at measurement points A and B versus time. Note that the distance between the measurement points A and B is 3145 μm.

It is apparent from FIGS. 13(b) to 13(d) that the cardiomyocytes cultured on the scaffold constituted by the gelatin nonwoven and the gelatin film beat in synchronization even in a wide region with a width of 3 mm or more because the concentrations of calcium ions in the cells changed at the same timing.

(2) Evaluation of Response against Isoproterenol

A calcium indicator was added to the culture medium at a mass ratio of 5/100 in the same manner as described above, and fluorescently stained calcium ions in the cardiomyocytes were observed after the 2-hour culture. After the observation, isoproterenol, which is a beta-stimulant used to treat bradycardia, atrioventricular block, and bronchial asthma, was added to the liquid culture medium at a concentration of 100 nM as a compound for accelerating the beating of the cardiomyocytes, and fluorescently stained calcium ions in the cardiomyocytes were observed 5 minutes later.

FIG. 14 shows the results illustrating the response against isoproterenol on the fourth day of the culture in Example 5. Adding isoproterenol at a concentration of 100 nM accelerated the change in the concentrations of intracellular calcium ions, and changed the beating rate. Specifically, the beating rate was 60 bpm before the addition, whereas the beating rate was 76 bpm after the addition.

As is clear from these results, comparing the beating rates of cardiomyocytes between before and after delivering a target compound into contact with the multicellular construct and/or the peak shapes of fluorescence intensities over time makes it possible to determine the effects of the target compound on the cardiomyocytes.

Example 6

Evaluation of Compound Using iPS Derived Cardiomyocytes 2

(1) Evaluation of Synchronous Beating of Cardiomyocytes

Cells were seeded in the same manner as in Example 4, except that a cell suspension obtained by suspending cells at a concentration of $2\times10^7$ cells/mL was used. A liquid culture medium was added 4 hours later to start cell culturing. On and after the fourth day of the culture, a calcium indicator (EarlyTox Cardiotoxicity Kit, Molecular Device) was added to the culture medium at a volume ratio of 5/100 to fluorescently stain calcium ions in the cardiomyocytes. The cell seeding amount per unit area of the scaffold was 6250 cells/mm$^2$.

Videos were captured at 20 FPS using a confocal imaging confocal imaging system (CellVoyager CQ1, Yokogawa Electric Corp.). Fluorescent areas in the obtained videos were recognized using an analysis software (Cell-Pathfinder, Yokogawa Electric Corp.), and signal waveforms of calcium ions in the cardiomyocytes were obtained by plotting the fluorescence intensities versus time.

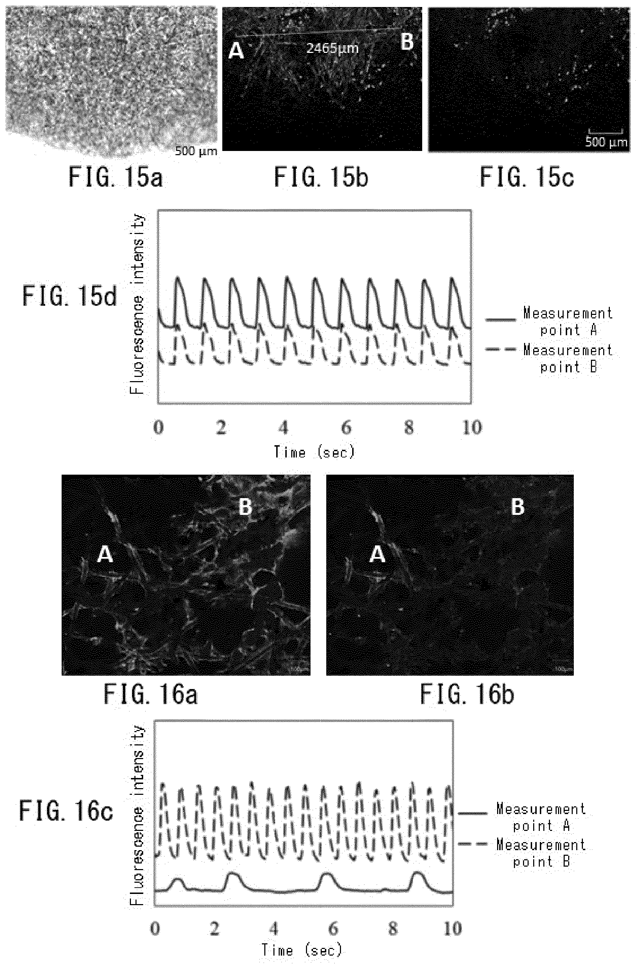

FIG. 15 shows the imaging evaluation results of calcium ions in the cells of the multicellular construct after the calcium indicator had been added on the fourth day of the culture. FIG. 15(a) is a bright field image, FIG. 15(b) is a fluorescence image of calcium ions in the cells observed 120 minutes after the addition of the calcium indicator, FIG. 15(c) is a fluorescence image of calcium ions in the cells observed 0.7 seconds after the image shown in FIG. 15(b) had been taken, and FIG. 15(d) is a graph obtained by plotting the fluorescence intensities at measurement points A and B versus time. Note that the distance between the measurement points A and B is 2465 $\mu$m.

It is apparent from FIGS. 15(b) to 15(d) that the multicellular construct, that is, the cardiomyocytes cultured on the scaffold constituted by the gelatin nonwoven and the gelatin film, beat in synchronization even in a wide region with a width of 2 mm or more because the concentrations of calcium ions in the cells changed at the same timing.

(2) Evaluation of Response against Arrhythmia-Inducing Compound

A calcium indicator was added to the culture medium at a volume ratio of 5/100 in the same manner as described above, and fluorescently stained calcium ions in the cardiomyocytes were observed after the 2-hour culture. Then, E-4031 (FUJIFILM Wako Pure Chemical Corporation), which is an inhibitor of delayed rectifier potassium current ($Ik_r$), was added to the liquid culture medium at a concentration of 900 nM as an arrhythmogenic compound, and fluorescence was observed 120 minutes after the addition.

FIG. 16 shows the results illustrating the response against E-4031 on the fourth day of the culture. FIG. 16(a) is a fluorescence image of intracellular calcium ions observed 120 minutes after the addition of E-4031, FIG. 16(b) is a fluorescence image of intracellular calcium ions observed 0.4 seconds after the image shown in FIG. 16(a) had been taken, and FIG. 16(c) is a graph obtained by plotting the fluorescence intensities at measurement points A and B versus time.

As is clear from FIGS. 16(a) to 16(c), when E-4031 was added to the multicellular construct, that is, the cardiomyocytes cultured on the scaffold constituted by the gelatin nonwoven and the gelatin film, the concentrations of calcium ions at different sites changed at different timings, and the synchronous beating was suppressed. It is confirmed from FIG. 16(c) that the beating behavior at the measurement point B was significantly different from that at the measurement point A, and arrhythmia occurred.

Example 7

Evaluation of Compound Using iPS Cells Derived Cardiomyocytes 3

(1) Production of Cardiomyocyte Multicellular Construct

Cells were seeded and cultured in the same manner as in Example 6, except that the cells were seeded such that the seeding amount per unit area of the scaffold was 6000 cells/mm$^2$.

(2) Structural Observation of Cardiomyocyte Multicellular Construct

On the third day and the seventh day of the culture, the scaffold containing the cardiomyocytes (cardiomyocyte multicellular Construct) was collected, fixed using 16% paraformaldehyde, and fluorescently stained. Structural observation was performed after the cell nuclei had been stained using 1mg/mL DMSO solution of bisbenzimide H33342 fluorochrome trihydrochloride (Nacalai Tesque Inc.), actin filaments had been stained using Alexa Fluor® 568 phalloidin (Invitrogen), and $\alpha$-actinin had been immunostained using Anti-$\alpha$-Actinin (Sarcomeric) antibody (Sigma) as a primary antibody.

(3) Calcium Imaging Evaluation of Response of Cardiomyocyte Multicellular Construct against Compound

On the sixth day of the culture, the cardiomyocyte multicellular construct was placed such that a surface containing more cells faced the bottom surface of a culture dish (IWAKI Multiwell Plate, Non-treated surface for suspension cell culture 6 Well), and was then left to stand for a day. On the seventh day of the culture, a calcium indicator (EarlyTox Cardiotoxicity Kit, Molecular Devices LLC) was added to the culture medium at a volume ratio of 25/100 to fluorescently stain calcium ions in the cardiomyocytes.

Each of the compounds listed in Table 4 below was cumulatively added to the target concentration, and a change in the concentration of calcium ions was observed to determine the response against the compound. A time-lapse fluorescence image was captured at 20 FPS using a confocal imaging confocal imaging system (CellVoyager CQ1, Yokogawa Electric Corp.) 10 minutes after the compound had been added at each concentration. The fluorescence intensities in the entire 10-fold magnified visual fields of the obtained images were plotted versus time using an analysis software CellPathfinder, Yokogawa Electric Corp.), and thus signal waveforms of calcium ions in the cardiomyocytes were obtained. Verapamil is a Ca channel antagonist having an $Ik_r$ inhibitory effect.

(4) Evaluation of Contractile Force of Cardiomyocyte Multicellular Construct

A bright field image of each specimen whose responses against the compounds listed in Table 4 below had been observed using the above-mentioned calcium ion indicator was captured at 20 FPS using a confocal imaging system (CellVoyager CQ1, Yokogawa Electric Corp.). The contractile force and the contraction-relaxation rate of the cardiomyocyte multicellular construct were evaluated from the obtained image using MUSCLEMOTION (Sala et al., Circ. Res., 2018), which is a plugin tool of ImageJ.

Table 4

| Compound | Concentration (nM) | | | | | | | |
|---|---|---|---|---|---|---|---|---|
| Isoproterenol | 0 | 1 | 3 | 10 | 30 | 100 | 300 | 1000 |
| E-4031 | 0 | 1 | 3 | 10 | 30 | 100 | 300 | 1000 |
| Verapamil | 0 | 1 | 3 | 10 | 30 | 100 | 300 | 1000 |

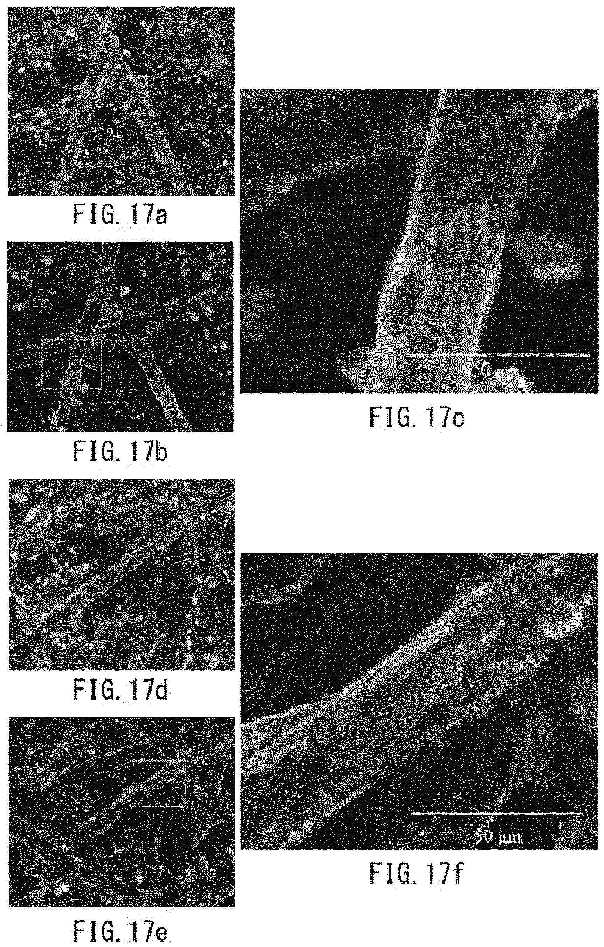

FIG. 17 shows the results of fluorescence staining of the cardiomyocyte construct in Example 7. FIG. 17(a) shows a stained image of cell nuclei and actin filaments on the third day of the culture, FIG. 17(b) shows an immunostained image of $\alpha$-actanin on the third day of the culture, and FIG. 17(c) is a partially enlarged image of FIG. 17(b). FIG. 17(d) shows a stained image of cell nuclei and actin filaments on the seventh day of the culture, FIG. 17(e) shows an immunostained image of $\alpha$-actanin on the seventh day of the culture, and FIG. 17(f) is a partially enlarged image of FIG. 17(e). The scale bars in FIG. 17 indicate 50 $\mu$m.

As is clear from the results of the phalloidin staining shown in FIG. 17(a), in the cardiomyocyte multicellular construct, actin filaments were present so as to cover the gelatin fibers included in the scaffold and were coupled to one another over a wide area on the third day of the culture, namely during a short-period of culture. As is clear from the results of the immunostaining of $\alpha$-actinin shown in FIGS. 17(b) and 17(c), sarcomere structures (striped pattern) included in cardiomyocytes were expressed and oriented in the longitudinal direction of the gelatin fiber. In addition, on the seventh day of the culture, the sarcomere structures grew and further thickened, and were more clearly oriented in the longitudinal direction of the gelatin fiber. It is known that the cardiomyocyte sarcomere structures are oriented in one direction in mature cardiomyocytes. With the cardiomyocyte multicellular construct, cardiomyocytes derived from iPS cells are oriented at an early stage, and thus structurally mature cardiomyocytes can be obtained. The sarcomere length in the sarcomere structure was about 2 $\mu$m, which is substantially the same as that of mature myocardium, and therefore, it is found that the cardiomyocytes were matured.

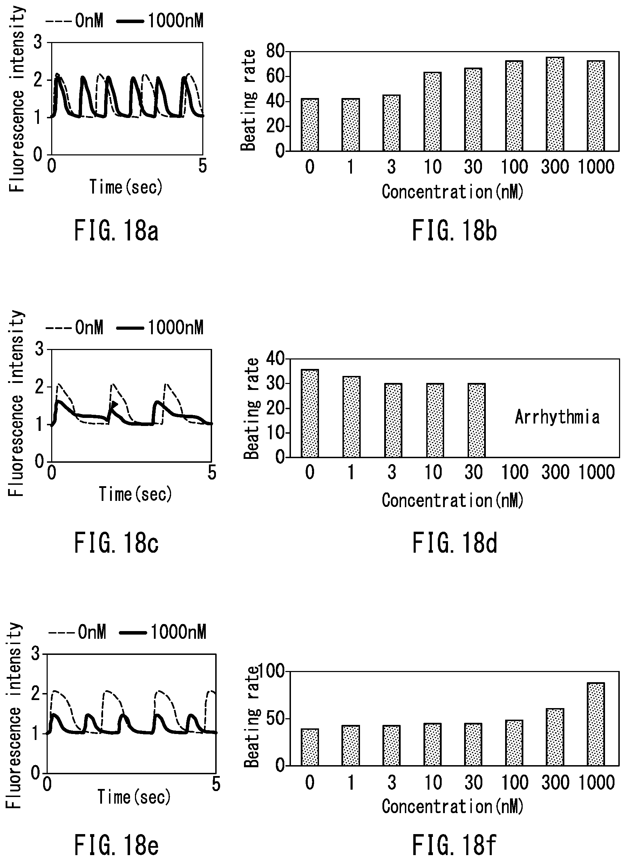

FIG. 18 shows graphs illustrating the responses against various compounds in Example 7. FIG. 18(a) shows the calcium ion signal waveforms before and after adding isoproterenol at a concentration of 1000 nM, and FIG. 18(b) shows the beating rates with various concentrations of isoproterenol. FIG. 18(c) shows the calcium ion signal waveforms before and with adding E-4031 at a concentration of 1000 nM, and FIG. 18(d) shows the beating rates for various concentrations of E-4031. FIG. 18(e) shows the calcium ion signal waveforms before and after adding verapamil at a concentration of 1000 nM, and FIG. 18(f) shows the beating rates for various concentrations of verapamil.

As is clear from FIG. 18, the cardiomyocyte multicellular construct exhibited different responses for compounds having different effects. When isoproterenol was added, the calcium ion signal waveform became sharper (a) and the beating rate increased (b) compared with those before the addition. When E-4031 was added, the calcium ion signal waveform became flattened (c), and the beating rate decreased compared with those before the addition. When E-4031 was added at a concentration of 100 nM or more, arrhythmia occurred (d). When verapamil was added, the calcium ion signal waveform became smaller (e) and the beating rate increased (f) compared with those before the addition.

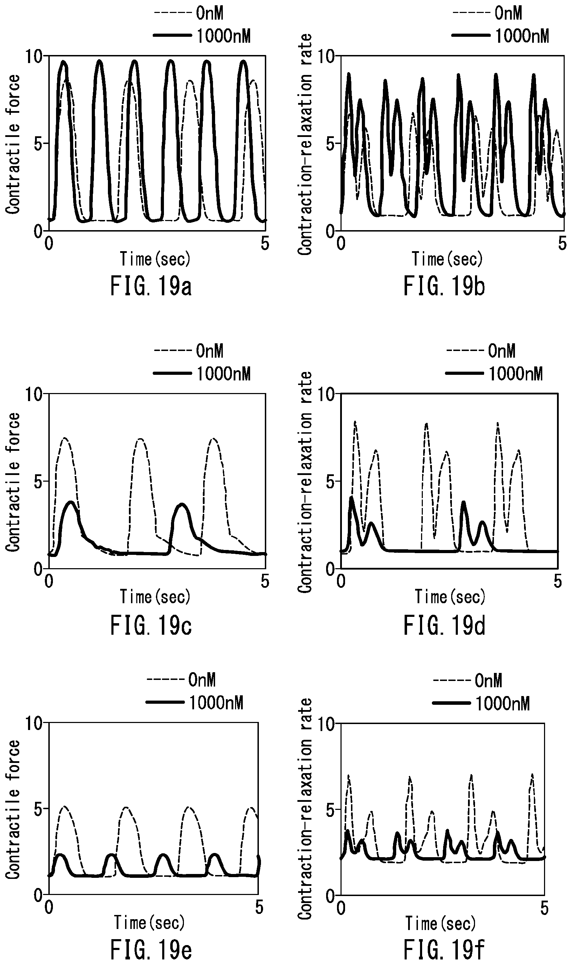

FIG. 19 shows graphs illustrating the responses against various compounds in Example 7. FIG. 19(a) shows the contractile forces before and after adding isoproterenol at a concentration of 1000 nM, and FIG. 19(b) shows the contraction-relaxation rates before and after adding isoproterenol at a concentration of 1000 nM. FIG. 19(c) shows the contractile forces before and after adding E-4031 at a concentration of 1000 nM, and FIG. 19(d) shows the contraction-relaxation rates before and after administering E-4031 at a concentration of 1000 nM. FIG. 19(e) shows the contractile forces before and after adding verapamil at a concentration of 1000 nM, and FIG. 19(f) shows the contraction-relaxation rates before and after adding verapamil at a concentration of 1000 nM.

As is clear from FIG. 19, the cardiomyocyte multicellular construct changed in the contractile force and the contraction-relaxation rate in different ways for compounds having different effects, and thus the responses against the compounds could be easily evaluated. When isoproterenol was added, the contractile force increased (a) and the contraction-relaxation rate also increased (b) compared with those before the addition. When E-4031 was added, the contractile force decreased (c) and the contraction-relaxation rate also decreased (d) compared with those before the addition. When verapamil was added, the contractile force decreased (e) and the contraction-relaxation rate also decreased (f) compared with those before the addition. The contractile force increased (the positive inotropic effect was exhibited) due to the addition of isoproterenol, which is a β-receptor stimulant, and therefore, it is found that the cardiomyocytes in the cardiomyocyte multicellular construct were matured.

Example 8

Evaluation of Compound Using Cardiomyocytes Derived from iPS Cells 4

(1) Production of Cardiomyocyte Multicellular Construct

Cells were seeded and cultured in the same manner as in Example 7, except that the cells were seeded such that the seeding amount per unit area of the scaffold was 8000 cells/mm$^2$.

(2) Calcium Imaging Evaluation of Response of Cardiomyocyte Multicellular Construct against Compound

Calcium ions in the cardiomyocytes were fluorescently stained in the same manner as in Example 7, except that the cardiomyocyte multicellular construct was placed such that a surface containing more cells faced the bottom surface of a culture dish on the day before the addition of the calcium indicator, and the calcium indicator was added during the sixth to tenth days of the culture.

Each of the compounds listed in Table 5 below (these are considered candidates as COVID-19 therapeutic drugs) was cumulatively added to the target concentration, and signal waveforms of calcium ions in the cardiomyocytes were obtained in the same manner as in Example 7.

(3) Evaluation of Contractile Force of Cardiomyocyte Multicellular Construct

The contractile force of the cardiomyocyte multicellular construct in each specimen whose responses against the compounds listed in Table 5 below had been observed using the above-mentioned calcium ion indicator was evaluated in the same manner as in Example 7.

Table 5

| Compound | Dose 0 (μM) | Dose 1 (μM) | Dose 2 (μM) | Dose 3 (μM) | Dose 4 (μM) |
|---|---|---|---|---|---|
| Azithromycin | 0 | 1 | 3 | 10 | - |
| Chloroquine | 0 | 0.3 | 1 | 3 | 10 |

(continued)

| Compound | Dose 0 ($\mu$M) | Dose 1 ($\mu$M) | Dose 2 ($\mu$M) | Dose 3 ($\mu$M) | Dose 4 ($\mu$M) |
|---|---|---|---|---|---|
| Hydroxychloroquine | 0 | 0.3 | 1 | 3 | 10 |
| Azithromycin (fixed to 3 $\mu$M) and chloroquine | 0 | 0.3 | 1 | 3 | 10 |
| Azithromycin (fixed to 3 $\mu$M) and hydroxychloroquine | 0 | 0.3 | 1 | 3 | 10 |

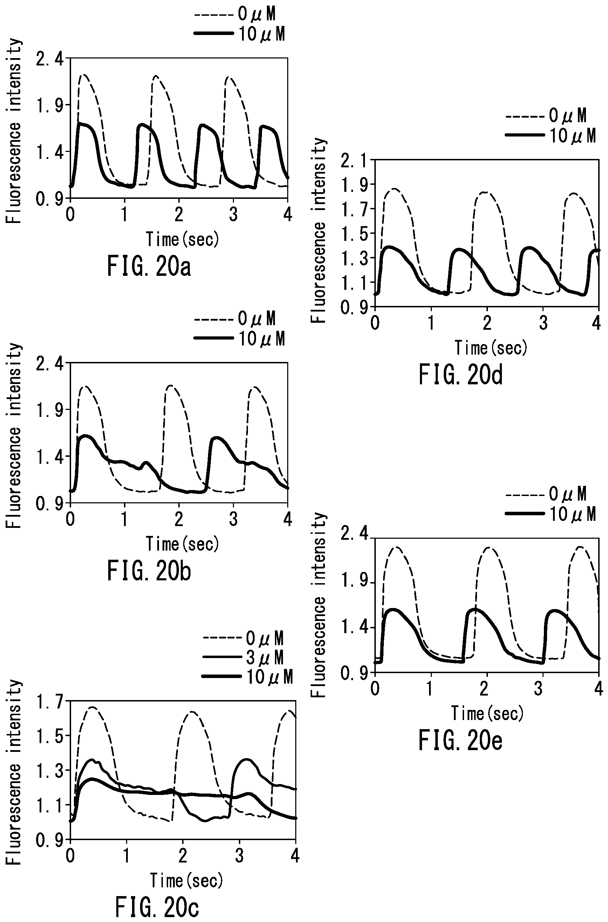

FIG. 20 shows graphs illustrating the responses against various compounds (calcium ion signal waveforms) in Example 8. FIG. 20(a) shows the results from the case of azithromycin, FIG. 20(b) shows the results from the case of chloroquine, FIG. 20(c) shows the results from the case of hydroxychloroquine, FIG. 20(d) shows the results from the case of a combination of azithromycin (3 $\mu$M) and chloroquine, and FIG. 20(e) shows the results from the case of a combination of azithromycin (3 $\mu$M) and hydroxychloroquine.

Table 6 below shows the ratios of specimens that exhibited the calcium ion signal waveform corresponding to arrhythmia when the compounds listed in Table 5 above were used in Example 8.

Table 6

| Drug | Dose 0 (%) | Dose 1 (%) | Dose 2 (%) | Dose 3 (%) | Dose 4 (%) |
|---|---|---|---|---|---|
| Azithromycin | 0 | 0 | 0 | 0 | 0 |
| Chloroquine | 0 | 0 | 0 | 20 | 40 |
| Hydroxychloroquine | 0 | 0 | 0 | 20 | 80 |
| Azithromycin (3 $\mu$M) and chloroquine | 0 | 0 | 0 | 0 | 20 |
| Azithromycin (3 $\mu$M) and hydroxychloroquine | 0 | 0 | 0 | 0 | 20 |

It is found from FIG. 20 and Table 6 that chloroquine and hydroxychloroquine increase the risk of arrhythmia, whereas using azithromycin together with chloroquine or hydroxychloroquine reduces the risk of arrhythmia.

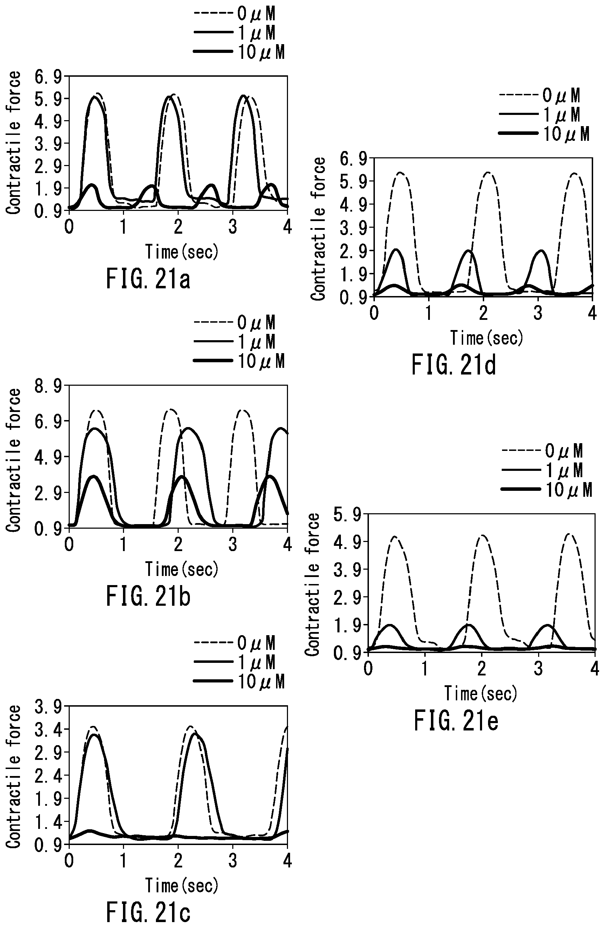

FIG. 21 shows graphs illustrating the responses against various compounds (contractile forces) in Example 8. FIG. 21(a) shows the results from the case of azithromycin, FIG. 21(b) shows the results from the case of chloroquine, FIG. 21(c) shows the results from the case of hydroxychloroquine, FIG. 21(d) shows the results from the case of a combination of azithromycin (3 $\mu$M) and chloroquine, and FIG. 21(e) shows the results from the case of a combination of azithromycin (3 $\mu$M) and hydroxychloroquine.

In Example 8, the compounds were added to the specimens at concentrations listed in Table 5 above, and the ratios of the specimens whose contractile force decreased to half of that before the addition of the compounds were determined and shown in Table 7 below.

Table 7

| Drug | Dose 0 (%) | Dose 1 (%) | Dose 2 (%) | Dose 3 (%) | Dose 4 (%) |
|---|---|---|---|---|---|
| Azithromycin | 0 | 0 | 0 | 100 | - |
| Chloroquine | 0 | 0 | 0 | 20 | 80 |
| Hydroxychloroquine | 0 | 0 | 0 | 20 | 100 |
| Azithromycin (3 $\mu$M) and chloroquine | 0 | 0 | 40 | 80 | 80 |
| Azithromycin (3 $\mu$M) and hydroxychloroquine | 0 | 0 | 60 | 100 | 100 |

It is found from FIG. 21 and Table 7 that when azithromycin and chloroquine or hydroxychloroquine are used together, the contractile force decreases at a lower concentration compared with the case where chloroquine or hydroxychloroquine is used alone, and a risk of the contractile impairment increases.

Example 9

Evaluation of Compound Using Cardiomyocytes Derived from iPS Cells 5

(1) Production of Cardiomyocyte Multicellular Construct

Cells were seeded and cultured in the same manner as in Example 3, except that Pluricyte ® (Ncardia) was used as cardiomyocytes derived from iPS cells, and the cells were seeded such that the seeding amount per unit area of the scaffold was 6000 cells/mm$^2$.

(2) Evaluation of Response of Cardiomyocyte Multicellular Construct against Compound Using Membrane Potential Indicator

On the seventh day of the culture, the cardiomyocyte multicellular construct was placed such that a surface containing more cells faced the bottom surface of a culture dish (IWAKI Multiwell Plate, Non-treated surface for suspension cell culture 6 Well), and was then left to stand for a day. On the eighth day of the culture, a membrane potential indicator (FluoVolt, Invitrogen) was added to the culture medium, and the membrane potential in the cardiomyocytes was measured through fluorescence staining after incubation at 37°C for 30 minutes.

E-4031 was cumulatively added to the culture solution such that the concentration thereof was 0, 1, 10, 100, and 1000 nM, and a change in the membrane potential was observed to determine the response. A time-lapse fluorescence image was captured at 20 FPS using a confocal imaging confocal imaging system (CellVoyager CQ1, Yokogawa Electric Corp.) 10 minutes after the compound had been added at each concentration. The fluorescence intensities in the entire 10-fold magnified visual fields of the obtained images were plotted versus time using an analysis software CellPathfinder, Yokogawa Electric Corp.), and thus signal waveforms of membrane potential in the cardiomyocytes were obtained.

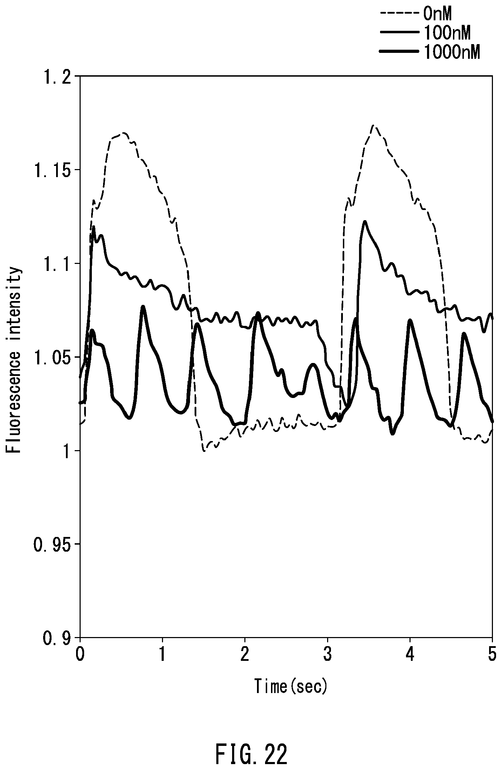

FIG. 22 shows the results of the observation of the response against the compound using the membrane potential indicator in Example 9. As is clear from FIG. 22, also in the case where the change in the membrane potential was observed using the membrane potential indicator, the prolonged waveforms were found due to the addition of E-4031, and thus arrhythmia could be evaluated.

It is found from these results that comparing the calcium ion signal waveforms, contractile forces, membrane potential signal waveforms, and the like of cardiomyocytes between before and after delivering a target compound into contact with the multicellular construct makes it possible to determine the effects of the compound on the cardiomyocytes (e.g., whether or not the compound has cardio-toxicity).

As is clear from these results, in one or more embodiments of the present invention, the multicellular construct can be used in, for example, a method for evaluating various characteristics of a compound such as metabolism, a pharmacological effect, and a toxic effect.

The present invention is not particularly limited, but preferably includes the following aspects.

[1] A multicellular construct that includes cells and a scaffold,

wherein the scaffold is constituted by a layered composite that includes a gelatin nonwoven containing gelatin as a main component, and a gelatin film containing gelatin as a main component and layered on one surface of the gelatin nonwoven, and
the cells are present in at least one of a region on the surface of the gelatin nonwoven and a region inside the nonwoven.

[2] The multicellular construct according to [1], wherein gelatin fibers included in the gelatin nonwoven have an average fiber diameter of 2 $\mu$m or more and 400 $\mu$m or less after swelling n and are welded to one another at some or all of fiber cross-points thereof, and the gelatin film is welded to some of the gelatin fibers included in the gelatin nonwoven.

[3] The multicellular construct according to [1] or [2], wherein the gelatin nonwoven has a thickness of 0.1 mm or more and 2.0 mm or less, and a basis weight of 10 g/m$^2$ or more and 600 g/m$^2$ or less.

[4] The multicellular construct according to any one of [1] to [3], wherein the gelatin film has a thickness of 0.80 $\mu$m or more and 3.20 $\mu$m or less.

[5] The multicellular construct according to any one of [1] to [4], wherein a ratio Tf/Tn of a thickness Tf of the gelatin film to a thickness Tn of the gelatin nonwoven is $7.5 \times 10^{-3}$ or less.

[6] The multicellular construct according to any one of [1] to [5], wherein the gelatin nonwoven and the gelatin film have undergone dehydrothermal crosslinking.

[7] The multicellular construct according to any one of [1] to [6], wherein the cells are present in both a region on the surface of the gelatin nonwoven and a region inside the nonwoven.

[8] The multicellular construct according to any one of [1] to [7], wherein the cells are one or more types of cells selected from the group consisting of stem cells; cancer cells; cardiomyocytes, neurons, hepatocytes, fibroblasts, endothelial cells, and epithelial cells that are derived from stem cells; and cardiomyocytes, neurons, hepatocytes, fibroblasts, endothelial cells, and epithelial cells that are derived from a living tissue.

[9] The multicellular construct according to any one of [1] to [8], wherein the cells include mature cardiomyocytes, and the mature cardiomyocytes are derived from cardiomyocytes differentiated from stem cells or cardiomyocytes differentiated from somatic cells.

[10] A method for manufacturing the multicellular construct according to any one of [1] to [9], including:

> a step of preparing a scaffold constituted by a layered composite that includes a gelatin nonwoven containing gelatin as a main component, and a gelatin film containing gelatin as a main component and layered on one surface of the gelatin nonwoven, and a culture vessel whose inner surface has not undergone a hydrophilization treatment;
> a step of arranging the scaffold in a swollen state inside the culture vessel whose inner surface is in a dry state with the gelatin film of the scaffold being in contact with an inner bottom surface of the culture vessel; and
> a step of dripping a cell suspension on the gelatin nonwoven of the scaffold to start culturing.

[11] The method for manufacturing the multicellular construct according to [10], wherein the cell suspension is dripped on the gelatin nonwoven of the scaffold and left to stand for a predetermined period of time, and then a liquid culture medium is added thereto to start cell culturing.

[12] The method for manufacturing the multicellular construct according to [10] or [11], wherein the cell suspension contains cardiomyocytes differentiated from stem cells or cardiomyocytes differentiated from somatic cells, and the cardiomyocytes are matured by culturing the cells for a predetermined period of time.

[13] A kit for producing the multicellular construct according to any one of [1] to [9], including:

> a scaffold constituted by a layered composite that includes a gelatin nonwoven containing gelatin as a main component, and a gelatin film containing gelatin as a main component and layered on one surface of the gelatin nonwoven; and a culture vessel whose inner surface has not undergone a hydrophilization treatment,
> wherein, during production of the multicellular construct, the scaffold in a swollen state is arranged inside the culture vessel whose inner surface is in a dry state with the gelatin film of the scaffold being in contact with an inner bottom surface of the culture vessel, and a cell suspension is dripped on the gelatin nonwoven of the scaffold.

[14] The kit for producing the multicellular construct according to [13], wherein the cell suspension contains cardiomyocytes differentiated from stem cells or cardiomyocytes differentiated from somatic cells, and the kit is used to produce mature cardiomyocytes.

[15] A method for evaluating a compound by evaluating characteristics of the compound, including:

> a step of delivering a compound into contact with the multicellular construct according to any one of [1] to [9]; and
> a step of determining whether or not physiological properties of the multicellular construct are changed due to the contact with the compound.

[16] The method for evaluating a compound according to [15], wherein the characteristics of the compound include one or more selected from the group consisting of metabolism, a pharmacological effect, and a toxic effect.

[17] The method for evaluating a compound according to [15] or [16], wherein the multicellular construct contains cardiomyocytes, and

in the determining step, one or more selected from the group consisting of intracellular calcium ion imaging, intracellular membrane potential imaging, and evaluation of contractile force of the multicellular construct are performed.

List of Reference Numerals

1  Warming tank
2  Spinning solution
3  Nozzle discharge outlet

4,6    Compressor
5    Fluid spout
7    Pressure fluid
8    Gelatin fiber
9    Gelatin nonwoven
10    Gelatin film
11    Winding roll
12    Heat-insulating container
20    Manufacturing apparatus

**Claims**

1. A multicellular construct comprising: cells; and a scaffold,

   wherein the scaffold is constituted by a layered composite that comprises a gelatin nonwoven containing gelatin as a main component, and a gelatin film containing gelatin as a main component and layered on one surface of the gelatin nonwoven, and
   the cells are present in at least one of a region on the surface of the gelatin nonwoven and a region inside the nonwoven.

2. The multicellular construct according to claim 1, wherein gelatin fibers included in the gelatin nonwoven have an average fiber diameter of 2 $\mu$m or more and 400 $\mu$m or less after swelling and are welded to one another at some or all of fiber cross-points thereof, and the gelatin film is welded to some of the gelatin fibers included in the gelatin nonwoven.

3. The multicellular construct according to claim 1 or 2, wherein the gelatin nonwoven has a thickness of 0.1 mm or more and 2.0 mm or less, and a basis weight of 10 g/m$^2$ or more and 600 g/m$^2$ or less.

4. The multicellular construct according to any one of claims 1 to 3, wherein the gelatin film has a thickness of 0.80 $\mu$m or more and 3.20 $\mu$m or less.

5. The multicellular construct according to any one of claims 1 to 4, wherein a ratio Tf/Tn of a thickness Tf of the gelatin film to a thickness Tn of the gelatin nonwoven is $7.5 \times 10^{-3}$ or less.

6. The multicellular construct according to any one of claims 1 to 5, wherein each of the gelatin nonwoven and the gelatin film has undergone dehydrothermal crosslinking.

7. The multicellular construct according to any one of claims 1 to 6, wherein the cells are present in both a region on the surface of the gelatin nonwoven and a region inside the nonwoven.

8. The multicellular construct according to any one of claims 1 to 7, wherein the cells are one or more types of cells selected from the group consisting of stem cells; cancer cells; cardiomyocytes, neurons, hepatocytes, fibroblasts, endothelial cells, and epithelial cells that are derived from stem cells; and cardiomyocytes, neurons, hepatocytes, fibroblasts, endothelial cells, and epithelial cells that are derived from a tissue.

9. The multicellular construct according to any one of claims 1 to 8, wherein the cells include mature cardiomyocytes, and the mature cardiomyocytes are derived from cardiomyocytes differentiated from stem cells or cardiomyocytes differentiated from somatic cells.

10. A method for manufacturing the multicellular construct according to any one of claims 1 to 9, comprising:

    a step of preparing a scaffold constituted by a layered composite that includes a gelatin nonwoven containing gelatin as a main component, and a gelatin film containing gelatin as a main component and layered on one surface of the gelatin nonwoven, and a culture vessel whose inner surface has not undergone a hydrophilization treatment;
    a step of arranging the scaffold in a swollen state inside the culture vessel whose inner surface is in a dry state with the gelatin film of the scaffold being in contact with an inner bottom surface of the culture vessel; and

a step of dripping a cell suspension on the gelatin nonwoven of the scaffold to start culturing.

11. The method for manufacturing the multicellular construct according to claim 10, wherein the cell suspension is dripped on the gelatin nonwoven of the scaffold and left to stand for a predetermined period of time, and then a liquid culture medium is added thereto to start cell culturing.

12. The method for manufacturing the multicellular construct according to claim 10 or 11, wherein the cell suspension contains cardiomyocytes differentiated from stem cells or cardiomyocytes differentiated from somatic cells, and the cardiomyocytes are matured by culturing the cells for a predetermined period of time.

13. A kit for producing the multicellular construct according to any one of claims 1 to 9, comprising:

a scaffold constituted by a layered composite that comprises a gelatin nonwoven containing gelatin as a main component, and a gelatin film containing gelatin as a main component and layered on one surface of the gelatin nonwoven; and a culture vessel whose inner surface has not undergone a hydrophilization treatment, wherein, during production of the multicellular construct, the scaffold in a swollen state is arranged inside the culture vessel whose inner surface is in a dry state with the gelatin film of the scaffold being in contact with an inner bottom surface of the culture vessel, and a cell suspension is dripped on the gelatin nonwoven of the scaffold.

14. The kit for producing the multicellular construct according to claim 13, wherein the cell suspension contains cardiomyocytes differentiated from stem cells or cardiomyocytes differentiated from somatic cells, and the kit is used to produce mature cardiomyocytes.

15. A method for evaluating a compound by evaluating characteristics of the compound, comprising:

a step of delivering a compound into contact with the multicellular construct according to any one of claims 1 to 9; and
a step of determining whether or not physiological properties of the multicellular construct are changed due to the contact with the compound.

16. The method for evaluating a compound according to claim 15, wherein the characteristics of the compound include one or more selected from the group consisting of metabolism, a pharmacological effect, and a toxic effect.

17. The method for evaluating a compound according to claim 15 or 16, wherein the multicellular construct contains cardiomyocytes, and
in the determining step, one or more selected from the group consisting of intracellular calcium ion imaging, cellular membrane potential imaging, and evaluation of contractile force of the multicellular construct are performed.

FIG. 1

FIG. 2

FIG. 3a

FIG. 3b

FIG. 4a    FIG. 4c    FIG. 4e

FIG. 4b    FIG. 4d    FIG. 4f

FIG. 5a

FIG. 5c

FIG. 5e

FIG. 5b

FIG. 5d

FIG. 5f

FIG. 6a

Outside scaffold
(No cell leakage)

Scaffold
(Gelatin
nonwoven side)

FIG. 6b

Outside scaffold
(Substantially
no cell leakage)

Scaffold
(Gelatin
nonwoven side)

FIG. 7a

FIG. 7c

FIG. 7e

FIG. 7b

FIG. 7d

FIG. 7f

FIG. 8a

FIG. 8c

FIG. 8e

FIG. 8b

FIG. 8d

FIG. 8f

FIG. 9a

Outside scaffold
(Many cells leaked)

Scaffold
(Gelatin
nonwoven side)

FIG. 9b

Outside scaffold
(Many cells leaked)

Scaffold
(Gelatin
nonwoven side)

FIG. 10a

Outside scaffold
(Many cells leaked)

Scaffold
(Gelatin
nonwoven side)

FIG. 10b

Outside scaffold
(Many cells leaked)

Scaffold
(Gelatin
nonwoven side)

FIG. 11a

FIG. 11b

FIG. 11c

FIG. 11d

FIG. 11e

FIG. 11f

FIG. 12a

FIG. 12b

FIG. 12c

FIG. 12d

FIG. 12e

FIG. 12f

FIG. 13a      FIG. 13b      FIG. 13c

FIG. 13d

FIG. 14

FIG. 15a  FIG. 15b  FIG. 15c

FIG. 15d

FIG. 16a  FIG. 16b

FIG. 16c

FIG. 17a

FIG. 17b

FIG. 17c

FIG. 17d

FIG. 17e

FIG. 17f

FIG. 18a

FIG. 18b

FIG. 18c

FIG. 18d

FIG. 18e

FIG. 18f

FIG. 19a

FIG. 19b

FIG. 19c

FIG. 19d

FIG. 19e

FIG. 19f

EP 4 063 477 A1

FIG. 20a

FIG. 20b

FIG. 20c

FIG. 20d

FIG. 20e

FIG. 21a

FIG. 21b

FIG. 21c

FIG. 21d

FIG. 21e

FIG. 22

FIG. 23

| | **INTERNATIONAL SEARCH REPORT** | | International application No. |
|---|---|---|---|
| | | | PCT/JP2020/042861 |

**A. CLASSIFICATION OF SUBJECT MATTER**

C12M  1/00(2006.01)i;  C12M  3/00(2006.01)i;  C12N  5/071(2010.01)i;  C12N 5/0735(2010.01)i;  C12N  5/074(2010.01)i;  C12N  5/077(2010.01)i;  C12N 5/079(2010.01)i;  C12N  5/09(2010.01)i;  C12N  1/00(2006.01)i;  C12Q 1/02(2006.01)i; C12N 11/02(2006.01)i

FI:      C12N5/071; C12N5/09; C12N5/074; C12N5/077; C12N5/079; C12M1/00 C; C12M3/00 A; C12Q1/02; C12N11/02; C12N1/00 A; C12N5/0735

According to International Patent Classification (IPC) or to both national classification and IPC

**B. FIELDS SEARCHED**

Minimum documentation searched (classification system followed by classification symbols)

C12M1/00;  C12M3/00;  C12N5/071;  C12N5/0735;  C12N5/074;  C12N5/077; C12N5/079; C12N5/09; C12N1/00; C12Q1/02; C12N11/02

Documentation searched other than minimum documentation to the extent that such documents are included in the fields searched

| | |
|---|---|
| Published examined utility model applications of Japan | 1922–1996 |
| Published unexamined utility model applications of Japan | 1971–2021 |
| Registered utility model specifications of Japan | 1996–2021 |
| Published registered utility model applications of Japan | 1994–2021 |

Electronic data base consulted during the international search (name of data base and, where practicable, search terms used)

JSTPlus/JMEDPlus/JST7580 (JDreamIII); CAplus/MEDLINE/EMBASE/BIOSIS/WPIDS (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2014-514942 A (UNIVERSITÄTSKLINIKUM FREIBURG) 26 June 2014 (2014-06-26) in particular, claims 1-16, paragraphs [0095]-[0103], examples, fig. 2 | 1, 2, 7, 8, 15, 16 |
| Y | in particular, claims 1-16, paragraphs [0095]-[0103], examples, fig. 2 | 1-17 |
| X | JP 2004-148014 A (NIPRO CORP.) 27 May 2004 (2004-05-27) in particular, claims 1-17, paragraphs [0066], [0071], examples | 1, 7, 8 |
| Y | in particular, claims 1-17, paragraphs [0066], [0071], examples | 1-17 |

☒  Further documents are listed in the continuation of Box C.      ☒  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 14 January 2021 (14.01.2021) | 26 January 2021 (26.01.2021) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

**EP 4 063 477 A1**

**INTERNATIONAL SEARCH REPORT**

| | International application No. |
|---|---|
| | PCT/JP2020/042861 |

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2018-023361 A (PANASONIC IP MANAGEMENT CO., LTD.) 15 February 2018 (2018-02-15) in particular, paragraphs [0050]-[0068] | 1-17 |
| Y | JP 2018-007608 A (PANASONIC IP MANAGEMENT CO., LTD.) 18 January 2018 (2018-01-18) in particular, paragraphs [0043]-[0049], fig. 4, 7 | 1-17 |
| Y | WO 2018/235745 A1 (THE JAPAN WOOL TEXTILE CO., LTD.) 27 December 2018 (2018-12-27) in particular, claims 1-20, examples, fig. 4 | 1-17 |
| Y | JP 2019-526255 A (THE UNIVERSITY OF QUEENSLAND) 19 September 2019 (2019-09-19) in particular, examples | 17 |
| A | in particular, examples | 1-16 |
| A | JP 2019-123955 A (JAPAN VILENE COMPANY, LTD.) 25 July 2019 (2019-07-25) entire text | 1-17 |
| A | JP 2010-284373 A (KB SEIREN, LTD.) 24 December 2010 (2010-12-24) entire text | 1-17 |
| A | WO 2016/068266 A1 (KYOTO UNIVERSITY) 06 May 2016 (2016-05-06) entire text | 1-17 |

Form PCT/ISA/210 (continuation of second sheet) (January 2015)

44

INTERNATIONAL SEARCH REPORT
Information on patent family members

International application No.

PCT/JP2020/042861

| Patent Documents referred in the Report | Publication Date | Patent Family | Publication Date |
|---|---|---|---|
| JP 2014-514942 A | 26 Jun. 2014 | US 2014/0112973 A1 in particular, claims 1-16, paragraphs [0103]-[0111], examples, fig. 2 WO 2012/136701 A1 EP 2508212 A1 CN 103648536 A | |
| JP 2004-148014 A | 27 May 2004 | US 2006/0029639 A1 in particular, claims 1-21, paragraphs [0070], [0075], examples WO 2004/039578 A1 EP 1577083 A1 CN 1705559 A | |
| JP 2018-023361 A | 15 Feb. 2018 | (Family: none) | |
| JP 2018-007608 A | 18 Jan. 2018 | (Family: none) | |
| WO 2018/235745 A1 | 27 Dec. 2018 | (Family: none) | |
| JP 2019-526255 A | 19 Sep. 2019 | US 2019/0203179 A1 in particular, examples WO 2018/035574 A1 EP 3504321 A1 | |
| JP 2019-123955 A | 25 Jul. 2019 | (Family: none) | |
| JP 2010-284373 A | 24 Dec. 2010 | (Family: none) | |
| WO 2016/068266 A1 | 06 May 2016 | US 2017/0319747 A1 entire text | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014196549 A

- WO 2018235745 A

**Non-patent literature cited in the description**

- **SALA et al.** *Circ. Res.,* 2018